(19) Europäisches Patentamt
European Patent Office
Office européen des brevets

(11) Veröffentlichungsnummer: **0 221 344**
**B1**

(12) # EUROPÄISCHE PATENTSCHRIFT

(45) Veröffentlichungstag der Patentschrift: **23.05.90**

(51) Int. Cl.[5]: **C07C 311/03**, C07C 311/14, C07C 311/16, C07C 311/37, A61K 31/18, A61K 31/63

(21) Anmeldenummer: **86113481.5**

(22) Anmeldetag: **01.10.86**

(54) **Neue Sulfonyl-phenylalkylamine, Verfahren zu ihrer Herstellung sowie Arzneimittel.**

(30) Priorität: **02.10.85 DE 3535167**

(43) Veröffentlichungstag der Anmeldung:
**13.05.87 Patentblatt 87/20**

(45) Bekanntmachung des Hinweises auf die Patenterteilung:
**23.05.90 Patentblatt 90/21**

(84) Benannte Vertragsstaaten:
**AT BE CH DE ES FR GB GR IT LI LU NL SE**

(56) Entgegenhaltungen:
**EP-A- 0 031 954**
**EP-A- 0 194 548**

**BULLETIN OF THE CHEMICAL SOCIETY OF JAPAN, Band 50, Nr. 10, Oktober 1977, Seiten 2780-2784, Tokyo, JP; N. TORIMOTO et al.: "Copper-initiated decomposition of N,N-dichloromethanesulfonamide"**

(73) Patentinhaber: **BOEHRINGER MANNHEIM GMBH, Sandhofer Strasse 116, D-6800 Mannheim 31(DE)**

(72) Erfinder: **Witte, Ernst-Christian, Dr. rer. nat., Beethovenstrasse 2, D-6800 Mannheim 1(DE)**
Erfinder: **Wolff, Hans-Peter, Dr. rer. nat., Rebenweg 24, D-6945 Hirschberg-Grosssachsen(DE)**
Erfinder: **Stegmeier, Karlheinz, Dr. rer. nat., Kirchbergstrasse 17, D-6148 Heppenheim(DE)**
Erfinder: **Pill, Johannes, Dr. rer. nat. Dr. med., In der Keitgasse 5, D-6906 Leimen 3(DE)**

EP 0 221 344 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft am Phenyl in 4-Stellung substituierte Sulfonyl-phenylalkylamine und ihre Derivate, Verfahren zu ihrer Herstellung sowie Arzneimittel, die diese Verbindungen enthalten.

In der Europaeischen Patentanmeldung Nr. 31954 sin Sulfonamide mit lipidsenkender und die Thrombozytenaggregation hemmender Wirkung beschrieben, die sich durch die allgemeine Formel I charakterisieren lassen,

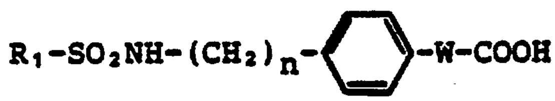

(I)

in welcher $R_1$ eine Aryl-, Aralkyl- oder Aralkenylgruppe, n die Zahlen 2 und 3, W eine Bindung oder eine unverzweigte oder verzweigte Alkylenkette mit 1-4 C-Atomen bedeuten, wobei letztere entweder gesaettigt ist oder eine Doppelbindung enthaelt.

Es wurde nun ueberraschenderweise gefunden, daß man Verbindungen mit lipidsenkender und thromboxan-$A_2$-antagonistischer Wirkung erhaelt, wenn man in Formel I den Rest -W-COOH durch eine Alkylkette mit 1-6 C-Atomen ersetzt, welche durch sauerstoffhaltige Funktionen substituiert sein kann.

Die vorliegende Erfindung betrifft daher neue Sulfonylphenylalkylamine der allgemeinen Formel II

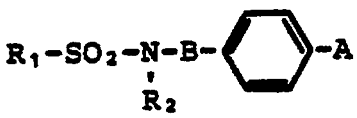

(II),

in welcher
$R_1$ eine niedere Alkylgruppe mit 1-6 C-Atomen,
einen Cycloalkylrest mit 3-7 C-Atomen,
einen Aralkyl-, Aralkenyl- oder Arylrest, wobei der Arylteil jeweils ein- oder mehrfach durch Halogen, $C_1$-$C_6$ Alkyl, $C_1$-$C_6$ Alkoxy, Hydroxyl, Trifluormethyl, Cyan, Nitro, Amino, $C_1$-$C_6$ Alkylamino, $C_2$-$C_{12}$Dialkylamino, $C_1$-$C_6$ Acylamino, $C_1$-$C_{16}$ Acyl oder Azid substituiert sein kann,
$R_2$ ein Wasserstoff,
eine Alkylgruppe mit 1-6 C-Atomen,
eine Acylgruppe, einen Aralkyl- oder Aralkenylrest darstellt, wobei der Arylteil jeweils ein- oder mehrfach durch Halogen, $C_1$-$C_6$ Alkyl, $C_1$-$C_6$ Alkoxy, Hydroxyl, Trifluormethyl, Cyan, Nitro, Amino, $C_1$-$C_6$ Alkylamino, $C_2$-$C_{12}$Dialkylamino, $C_1$-$C_6$ Acylamino, $C_1$-$C_{16}$Acyl oder Azid substituiert sein kann,
B eine unverzweigte oder verzweigte Alkylenkette mit maximal 4 C-Atomen und
A eine Alkyl- oder Alkenylgruppe mit 1-6 C-Atomen, eine $C_1$-$C_6$ Formylalkylgruppe, eine $C_1$-$C_6$ Hydroxyalkylgruppe oder einen Rest -D-$R_3$ darstellt, in dem D eine

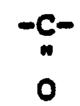

oder

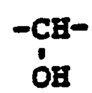

Gruppe und $R_3$ Wasserstoff, einen $C_1$-$C_5$ Alkyl-, einen $C_1$-$C_5$ Hydroxyalkyl- oder einen $C_1$-$C_5$ Alkylcarbonsäure-Rest und ggf. deren Lactone bedeuten,
darstellen, sowie deren pharmakologisch verträgliche Salze, mit Ausnahme der Verbindung Benzol-sulfonsäure-(4-acetyl-phenethylamid).

Benzolsulfonsäure-(4-acetyl-phenethylamid) ist in dem europäischen Patent 31 954 als Zwischenprodukt zur Herstellung der dort beanspruchten Carbonsäure ohne Angabe einer pharmakologischen Wirkung beschrieben.

Die neuen Verbindungen der allgemeinen Formel II zeigen eine ausgezeichnete antagonistische Wirkung gegenueber Thromboxan $A_2$ sowie gegen Prostaglandin-endoperoxide. Sie inhibieren die Aggregation von Blutplaettchen und verhindern die Konstriktion der glatten Muskulatur sowie die Bronchokonstriktion. Sie sind außerdem wertvolle Heilmittel zur Behandlung pathologischer Veränderungen der Nierenfunktion.

Diese Eigenschaften machen sie zu wertvollen Heilmitteln zur Behandlung z.B. von cardiovaskulaeren Erkrankungen und von Asthma und zur Prophylaxe einer Schocklunge. Sie koennen weiterhin verwendet werden bei Organtransplantationen und Nierendialyse und sind geeignet, Rezidive bei Magengeschwueren zu verhindern. Eine besondere Bedeutung liegt in der Moeglichkeit, thrombotische Prozesse guenstig zu beeinflussen oder zu verhindern. Sie sind zur Behandlung peripherer arterieller Verschlußkrankheiten geeignet und koennen z.B. gegen cerebrale ischaemische Zustaende eingesetzt werden.

Zusätzlich vermögen sie die Verwertung von Acetat zur Cholesterin-Biosynthese zu hemmen und eignen sich daher auch zur Behandlung von Fettstoffwechselerkrankungen.

Ist $R_1$ eine Alkylgruppe, so seien darunter unverzeigte oder verzweigte Alkylgruppen mit 1-6 C-Atomen verstanden. Bervorzugt sind die Methyl-, Ethyl- und die Hexylgruppe.

Als Cycloalkylreste für $R_1$ kommen beispielsweise die Cyclopropyl-, Cyclopentyl- und Cyclohexylreste in Frage.

Als "Arylrest", allein oder in Verbindung mit einer Alkyl- oder Alkylenkette, sind in allen Fällen aromatische Kohlenwasserstoffe mit 6-14 C-Atomen, insbesondere der Phenyl-, der Biphenylyl-, der Naphthyl- und der Fluorenylrest zu verstehen. Diese Arylreste können in allen möglichen Positionen einoder mehrfach substituiert sein, wobei als Substituenten Halogen, $C_1$-$C_6$ Alkyl, $C_1$-$C_6$ Alkoxyl, Hydroxyl, Trifluormethyl, Cyan, Nitro, Amino, $C_1$-$C_6$ Alkylamino, $C_2$-$C_{12}$ Dialkylamino, $C_1$-$C_6$ Acylamino, $C_1$-$C_6$ Acyl und Azid in Frage kommen.

Bevorzugt in diesem Sinne ist beispielsweise die Methoxy-, Ethoxy-, Methyl-, Ethyl-, Propyl-, tert.-Butyl-, Acetylamino-, Acetyl-, Methylamino-, Dimethylamino-, Ethylamino- und Diethylaminogruppe.

Als Aralkylreste $R_1$ kommen solche in Frage, deren geradkettiger oder verzweigter Alkylenanteil 1-5 Kohlenstoffatome enthält. Bevorzugte Aralkylreste $R_1$ sind der Phenethyl- und der 4-Chlor-phenethylrest.

Unter Aralkenylresten $R_1$ sind solche zu verstehen, deren Alkenylenanteil 2-3 Kohlenstoffatome enthält. Hier sind bevorzugt der Styrylrest und der 4-Chlor-styryl-rest.

Unter Halogen ist Fluor, Chlor und Brom zu verstehen.

Als Alkylgruppen $R_2$ kommen geradkettige oder verzweigte mit 1-6 C-Atomen infrage, bevorzugt ist die Methylgruppe. Unter einer Aralkylgruppe $R_2$ seien solche verstanden, deren Alkylenanteil 1-5 C-Atome enthaelt. Bevorzugte Aralkylreste sind Benzyl, 4-Chlor-benzyl, Phenethyl und 4-Chlor-phenethyl.

Unter Aralkenyl $R_2$ sind Gruppen zu verstehen, deren Alkenylenanteil 3-4 C-Atome enthaelt, wobei bevorzugt an Cinnamyl und 4-Chlor-cinnamyl gedacht wird.

Die Acylreste leiten sich von aliphatischen Carbonsäuren mit 2-16 C-Atomen, von araliphatischen und von aromatischen Carbonsäuren ab. Bevorzugte Acylgruppen sind Acetyl, Isobutyroyl, Cinnamoyl, Benzoyl, 4-Chlor-benzoyl und 4-Aminobenzoyl sowie n-Octanoyl und n-Hexadecanoyl.

B bedeutet eine Bindung oder eine 1-4 C-Atome enthaltende Alkylenkette. Bevorzugt sind die folgenden Alkylenketten:

$$-CH_2-,\ -(CH_2)_2-\ \text{und}\ -(CH_2)_3-\ \text{sowie}$$

$$\underset{\displaystyle CH_3}{-\overset{}{CH}-}\ \text{und}\ -CH_2-\underset{\displaystyle CH_3}{CH}-.$$

A wird definiert als Alkylkette mit 1-6 C-Atomen, die durch Sauerstoff enthaltende Funktionen wie Oxo- oder Hydroxygruppen und ggf zusaetzliche Carboxylgruppen substituiert sein kann.

Bevorzugt sind die nachstehend aufgefuehrten Gruppen A:

1. A = eine unverzweigte oder verzweigte gesaettigte oder einfach ungesaettigte Alkylkette mit 2-6 C-Atomen, bevorzugt Ethyl, n-Propyl, n-Butyl sowie Propenyl.

2. A = eine unverzweigte oder verzweigte gesaettigte Alkylkette, die endstaendig eine Hydroxygruppe traegt, insbesondere solche der Formel

$$-(CH_2)_p-OH$$

in welcher p die Zahlen 1 bis 6 darstellen kann.

3. A = eine Acylgruppe -C0-$R_3$, wobei

a) $R_3$ = H (Formylrest) oder

b) $R_3$ = ein unverzweigter oder verzweigter gesaettigter Alkylrest mit 1-5 C-Atomen; bevorzugt sind Methyl, Ethyl, n-Propyl und n-Butyl.

c) $R_3$ = ein unverzweigter oder verzweigter gesaettigter Alkylrest mit 1-5 C-Atomen, der endstaendig eine Hydroxygruppe traegt. Bevorzugt sind Verbindungen des Typs

$$R_3 = -(CH_2)_{p-1}-OH$$

mit p = 3, 4 und 5.

d) $R_3$ = eine unverzweigte oder verzweigte Alkylkette mit endstaendiger Carboxylfunktion, wobei bevorzugt sind Verbindungen der Form

$$R_3 = -(CH_2)_{p-2}-COOH$$

mit p = 2 bis 6, insbesondere aber p = 4 bis 6

4. A = eine Gruppe

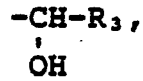

wobei $R_3$ die oben angegebenen Bedeutungen hat.

Fuer den Fall, daß $R_3$ ein wie unter 3 definierter Alkylrest ist, seien auch hier Methyl, Ethyl, n-Propyl und n-Butyl bevorzugt.

Ist $R_3$ dagegen eine Alkylgruppe mit endstaendiger Hydroxygruppe, so sind bevorzugt Verbindungen mit

$$R_3 = -(CH_2)_{p-1}-OH,$$

wobei p = 3, 4 oder 5 bedeutet.

Stellt $R_3$ eine Alkylgruppe mit endstaendiger Carboxylfunktion dar, so werden bevorzugt solche Verbindungen, in denen

$$R_3 = -(CH_2)_{p-2}-COOH$$

bedeutet, wobei p die Werte 4 oder 5 hat.

Besonders bevorzugt sind Verbindungen der allgemeinen Formel II, in welcher,

$R_1$ eine Methyl-, Ethyl- oder n-Hexylgruppe, einen Cyclohexylrest, einen Phenethyl- oder Styrylrest, in denen der Phenylteil durch Halogen substituiert sein kann, einen Phenylrest, der ggf. durch Halogen, Methyl, i-Propyl, Trifluormethyl, Methoxy, Hydroxy, Cyano, Nitro, Azid, Acetyl oder Acetylamino substituiert ist, eine Naphthylgruppe oder eine Biphenylgruppe, die durch Halogen substituiert sein kann, darstellt,

$R_2$ Wasserstoff, eine Methyl-, Acetyl-, Octanoyl-, Hexadecanoylgruppe, einen ggf. durch Halogen substituierten Benzoylrest, oder eine Benzyl-, Phenethyl- und Cinnamylgruppe, deren Phenylteil durch Halogen substituiert sein kann, bedeutet,

B eine Methylen-, Ethylen- oder Propylengruppe ist und

A eine Ethyl-, Propyl-, Butyl- oder Pentylgruppe, die ggf. ein oder zweifach durch Hydroxy, Carboxyl, Formyl, Acetyloxy und Benzoyloxy substituiert ist, eine Hydroxymethyl-, Formyl-, Propenyl-, Acetylvinylgruppe oder einen Acetyl-, Propionyl- und Butyrylrest, die jeweils durch Hydroxy, Carboxy, Ethoxycarbonyl und Methoxycarbonyl substituiert sein können, darstellt,

sowie deren Ester, Amide oder Lactone, sofern diese Verbindungen eine Carboxylgruppe oder eine Hydroxy- und Carboxylgruppe enthalten.

In allen Faellen soll der Begriff "Carboxylfunktion" auch Ester und Amide solcher Carbonsäuren einschließen.

Als Ester kommen solche mit niederen einwertigen Alkoholen (wie z.B. Methanol oder Ethanol) oder mit mehrwertigen Alkoholen (wie z.B. Glycerin) infrage, es seien aber auch solche Alkohole eingeschlossen, die noch andere funktionelle Gruppen enthalten, wie z.B. Ethanolamin.

Beansprucht werden auch die "inneren Ester" der Hydroxycarbonsaeuren geeigneter Kettenlaenge, die Lactone.

Fuer den Fall, daß die Funktion A asymmetrische Kohlenstoffatome enthaelt, so seien sowohl die reinen optischen Isomeren als auch deren Gemische/Razemate umfaßt. Enthaelt der Rest A Doppelbindungen, so werden hier die reinen E- und Z-Isomeren sowie auch deren Gemische beansprucht.

Die Herstellung der Verbindungen der allgemeinen Formel II ist dadurch gekennzeichnet, daß man

a) ein Amin der allgemeinen Formel III

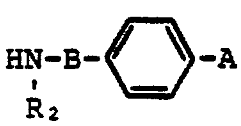

(III),

in welcher $R_2$, B und A die oben angegebene Bedeutung haben,

in an sich bekannter Weise mit einer Sulfonsaeure der allgemeinen Formel IV

$$R_1-SO_2OH \quad (IV),$$

in welcher $R_1$ hier und in allen folgenden Beispielen die oben angegebene Bedeutung hat, bzw. einem Derivat derselben umsetzt.

Anstelle der freien Amine III kann man auch deren Salze einsetzen.

b) ein Sulfonamid der allgemeinen Formel V

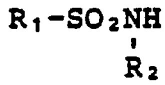 (V),

mit einer Verbindung der allgemeinen Formel VI

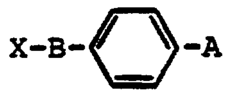 (VI),

zur Umsetzung bringt. X soll hier und in allen folgenden Erlaeuterungen eine reaktive Gruppe symbolisieren.
Fuer den Fall, daß A Hydroxygruppen enthalten soll, verwendet man in manchen Faellen vorteilhaft eine solche Verbindung VI, die an der Stelle der Hydroxygruppe eine Oxogruppe oder eine Esterfunktion (oder ggf. beide) enthaelt. Diese Gruppen werden im Anschluß an die erfolgte Reaktion zwischen V und VI zur Hydroxylfunktion reduziert.
c) Zur Herstellung von Verbindungen II, in denen $R_2$ die oben angegebene Bedeutung hat, jedoch kein Wasserstoffatom darstellt, kommt man auch durch nachtraegliches Einfuehren von $R_2$, indem man eine Verbindung der allgemeinen Formel II a

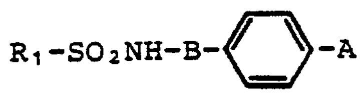 (II a),

mit einer Verbindung der allg. Formel VII

$R_2$-X (VII)

in der $R_2$ kein Wasserstoffatom darstellt,
umsetzt.
Bei Anwesenheit von Hydroxygruppen im Rest A gilt das unter b) Gesagte sinngemaeß.
d) Zur Einfuehrung von Hydroxygruppen in den Rest A kommen folgende Verfahren infrage:

1. Reduktion einer Carbonylgruppe
2. Reduktion einer Carbonsaeure- oder einer Carbonsaeureester-funktion.
3. Gleichzeitige Reduktion beider.

e) Die Einfuehrung von Doppelbindungen in den Rest A erfolgt, indem man aus einer in A enthaltenen Gruppe VIII

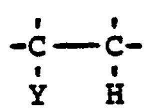 (VIII),

in welcher Y die Bedeutung Halogen oder Hydroxyl oder eine funktionell abgewandelte Hydroxylgruppe darstellt, HY abspaltet.
$e_2$) Eine weitere Moeglichkeit besteht in der Umsetzung von Oxoverbindungen mit geeigneten phosphororganischen Verbindungen im Sinne (modifizierter) Wittig-Reaktionen. Als Ausgangsmaterial dienen in diesem Falle Verbindungen der allg. Formel II, in denen

α) 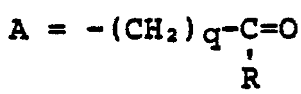 bzw.

β) $A = -(CH_2)_q-PO(O-Alk)_2$ bzw.

γ) $A = -(CH_2)_q-\overset{+}{P}R_3Hal^-$ bedeuten.

f) Durch Hydrieren der nach e) erhaltenen Verbindungen mit ungesaettigtem Rest A erhaelt man analoge

Verbindungen mit gesaettigter Kohlenstoffkette. Derartige Verbindungen lassen sich auch durch Reduktion von in A enthaltenen Ketogruppen darstellen.

Als reaktive Derivate der Sulfonsaeuren IV kommen insbesondere die Halogenide sowie die Ester infrage. Die Umsetzungen der Sulfonsaeurehalogenide mit Verbindungen der allgemeinen Formel III erfolgen zweckmaessig unter Zusatz eines saeurebindenden Mittels, wie z.B. Alkaliacetat, Natriumhydrogencarbonat, Natriumcarbonat, Natriumphosphat, Calciumoxid, Calciumcarbonnat oder Magnesiumcarbonat. Diese Funktion koennen aber auch organische Basen wie z.B. Pyridin oder Triethylamin uebernehmen, wobei als inertes Loesungsmittel z.B. Ether, Benzol, Methylenchlorid, Dioxan oder ein Ueberschuss des tertiaeren Amins dient.

Bei Einsatz anorganischer Saeurebinder verwendet man als Reaktionsmedium z.B. Wasser, waessriges Ethanol oder waessriges Dioxan.

Zur Alkylierung der Sulfonamide V verwendet man Verbindungen VI, in denen X ein Halogenatom wie Chlor oder Brom darstellt, bevorzugt aber solche, in denen X eine Arylsulfonyloxygruppe darstellt, als Alkylierungsmittel dienen also bevorzugt Arylsulfonsaeurealkylester, eine Methode, die in ihrer Anwendung auf Sulfonsaeureamide z.B. bei Klamann et al., Monatshefte fuer Chemie Bd. 83 (1952), S. 871 beschrieben ist. Die Umsetzungen erfolgen in alkalischem Milieu. Bevorzugt ist als Reaktionsmedium heiße, konzentrierte Sodaloesung. Ist dagegen X ein Halogenatom, so setzt man ein Alkalisalz des Sulfonamids V, z.B. das Natriumsalz, mit VI (X = Chlor oder Brom) in polaren Loesungsmitteln wie z.B. Dimethylformamid um. Um eine Disubstitution des Sulfonamids V zu vermeiden, wird V zweckmaeßig im Ueberschuß eingesetzt.

Soll im Anschluß an die Sulfonamidbildung eine Gruppe $R_2$ eingefuehrt werden, so geschieht dies durch Umsetzen einer Verbindung II, in der $R_2$ = H bedeutet, mit einem Saeurehalogenid, wenn $R_2$ eine Acylgruppe darstellt.

Fuer alle anderen Bedeutungen von $R_2$ setzt man ein Halogenid (Chlorid oder Bromid) der allg. Formel VII ein, wobei unter den oben angegebenen Bedingungen gearbeitet wird.

Die Acylierung des Sulfonamids erfolgt in einem inerten Loesungsmittel wie Ether oder Methylenchlorid, als saeurebindendes Agens verwendet man bevorzugt organische Basen wie Pyridin oder Triethylamin.

Die im Anschluß an die Sulfonamidbildung und ggf. nach Einfuehrung einer Gruppe $R_2$ moeglichen Umwandlungen im Rest A lassen sich wie folgt beschreiben:

Zur Umwandlung einer Carbonylgruppe in eine Hydroxygruppe sind hier alle gaengigen Verfahren einsetzbar. Bevorzugt ist die Reduktion mit komplexen Borhydriden, z.B. mit Natriumborhydrid, wobei protische Loesungsmittel wie Wasser, (waeßrige) Alkohole oder waeßriges Dioxan als Reaktionsmedium dienen. Bei Abwesenheit anderer reduzierbarer Gruppen kann die Reduktion auch mit komplexen Aluminiumhydriden wie $LiAlH_4$ durchgefuehrt werden, wobei hier aprotische Loesungsmittel wie Ether, THF oder Dioxan als Reaktionsmedium dienen. Die Carbonylreduktion kann aber auch mit katalytisch angeregtem Wasserstoff erfolgen, z.B. mit $H_2$/Raney-Nickel oder durch Umsetzen mit Nickel-Aluminium-Legierung in waeßrigem Alkali.

Zur Reduktion der Carboxylfunktion sind alle gaengigen Reduktionsmittel geeignet, z.B. komplexe Hydride wie Lithium-aluminium-hydrid oder Boran-addukte wie z.B. $BH_3$ . THF. Die Reduktion kann aber auch vorteilhaft durch Reduzieren eines Derivates der Carbonsaeure, z.B. eines gemischten Anhydrids aus der Carbonsaeure und einem Kohlensaeurehalbester, erfolgen. Als Reduktionsmittel verwendet man hier bevorzugt komplexe Borhydride wie z.B. $NaBH_4$ in protischen Loesungsmitteln.

Zur Reduktion geeignete Derivate der Carbonsaeuren sind z.B. auch deren Ester, welche sich nach literaturueblichen Methoden zu primaeren Alkoholen umsetzen lassen. Bevorzugte Reduktionsmittel sind auch hier komplexe Aluminiumhydride wie z.B. Lithiumalanat.

Soll die Carboxylfunktion reduziert werden, ohne daß eine gleichzeitig in A befindliche Oxogruppe mitreduziert wird, so ist letztere z.B. durch Ketalisierung intermediaer zu schuetzen. Solche Hydroxy-ketone sind auch darstellbar, indem man sowohl die Ketogruppe als auch die Carboxylfunktion reduziert (wobei man die ebenfalls beanspruchten Diole erhaelt) und anschließend die sekundaere Hydroxyfunktion selektiv zur Ketofunktion oxidiert. Hierzu geeignet ist z.B. aktives Mangandioxid.

Zur Einfuehrung von Doppelbindungen in den Rest A eignen sich alle gaengigen Eliminierungsreaktionen:

Abspaltung von Wasser aus Hydroxyverbindungen (saeurekatalysiert, durch Erwaermen mit z.B. Schwefelsaeure oder 85proz. Phosphorsaeure); Abspaltung von Halogenwasserstoff aus Halogeniden und von Sulfonsaeuren aus Sulfonyloxyverbindungen (z.B. Tosyloxy) mittels starker Basen wie Kalium-tert.-butanolat oder DBU, und schließlich die Abspaltung von Essigsaeure aus Acetoxyverbindungen und Xanthogensaeure aus Xanthogenaten durch Erhitzen.

Zum Aufbau von Gruppen A, die eine Doppelbindung enthalten, eignet sich auch die Kondensation von zwei Komponenten, deren eine Oxogruppe, die andere dagegen eine Phosphoniumsalz-Gruppe (Wittig-Reaktion) oder eine Phosphonat-Gruppe (Wittig-Horner-Emmons-Reaktion) enthalten. Diese Reaktionen koennen in verschiedensten Loesungsmitteln wie z.B. Wasser, Methanol DMF, Ethylenglykol oder Glykolethern durchgefuehrt werden und verlaufen in Gegenwart von Basen wie z.B. Natriumcarbonat, Natriumalkoholaten, Natriumhydrid oder Butyllithium, wobei je nach Reaktionsbedingungen cis- oder trans-Isomere oder Gemische beider entstehen koennen.

Zur Herstellung von Verbindungen, in denen A eine gesaettigte Alkylkette ist, kommt man durch Hydrierung von olefinischem A. Die Hydrierung wird vorzugsweise bei Normaldruck oder bei erhoehtem Druck in Gegegenwart von Metallkatalysatoren wie z.B. Palladium oder Platin in Loesungsmitteln wie z.B. Essigsaeure oder in niederen Alkoholen vorgenommen.

Weiterhin bevorzugt ist die Reduktion von Verbindungen, die im Rest A eine Hydroxy- oder eine Oxogruppe enthalten. Die Reduktion von Hydroxygruppen enthaltenden Verbindungen geschieht in Gegenwart starker Saeuren wie z.B. einer Spur Perchlorsaeure mittels Wasserstoff mit Hilfe von Palladium- oder Platinkatalysator. Zur Reduktion einer Oxogruppe eignen sich Zahlreiche Verfahren. Die Reduktion kann z.B. nach Clemmensen mittels Zink/Salzsaeure erfolgen, oder man bildet aus dem Keton zunaechst ein Tosylhydrazon und reduziert dieses. Bevorzugt ist aber auch hier die Reduktion mittels katalytisch angeregtem Wasserstoff unter den oben angegebenen Bedingungen.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylgukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel II in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel II können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

Beispiel 1

4-Chlor-benzolsulfonsaeure-(4-acetyl-phenethylamid)

Man ruehrt eine Suspension aus 10.0 g (50 mmol) 4-Acetyl-phenethylamin-hydrochlorid, 80 mL Methylenchlorid und 12.7 g (125 mmol) Triethylamin ca. 30 min lang und tropft dann bei 0°C langsam eine Loesung aus 11.8 g (56 mmol) 4-Chlor-benzolsulfonylchlorid und 50 mL Methylenchlorid zu. Nach 2 h Ruehren bei Raumtemperatur wird mit Methylenchlorid verduennt, mit Wasser, verd. HCl und wieder mit Wasser gewaschen, dann mit $Na_2SO_4$ getrocknet und eingedampft. Das Rohprodukt kristallisiert man zweimal aus Ethanol um.

Ausb. 11.7 g (69 % d.Th.), Schmp. 97-99°C.

In Analogie dazu werden durch Umsetzen verschiedener Sulfonsaeurechloride mit den jeweils notwendigen Aminkomponenten hergestellt:

2) 4-<4-[2-(Benzolsulfonylamino)ethyl]phenyl>-4-oxo-butansaeure-ethylester
Ausb. 91 % d.Th., Schmp. 77°C (Toluol + Heptan)

3) 4-Chlor-benzolsulfonsaeure-(4-acetyl-benzylamid)
Ausb. 41 % d.Th., Schmp. 132-133°C (Toluol).

4) Benzolsulfonsaeure-(4-acetyl-phenethylamid)
Die Umsetzung erfolgte hier in reinem Pyridin.
Ausb. 42 % d. Th., Schmp. 140-141°C (Ethanol).

5) 3,4-Dichlor-benzolsulfonsaeure-(4-acetyl-phenethylamid)
Ausb. 80 % d. Th., Schmp. 132-133°C (Toluol).

6) 4-Brom-benzolsulfonsaeure-(4-acetyl-phenethylamid)
Ausb. 91 % d. Th., Schmp. 130-131°C (Toluol).

7) 4-Fluor-benzolsulfonsaeure-(4-acetyl-phenethylamid)
Ausb. 72 % d. Th., Schmp. 106-107°C (Ethylacetat).

8) 4-Methoxy-benzolsulfonsaeure-(4-acetyl-phenethylamid)
Ausb. 86 % d. Th., Schmp. 122-123°C (Isopropanol).
9) 4-Isopropyl-benzolsulfonsaeure-(4-acetyl-phenethylamid)
Ausb. 66 % d. Th., Schmp. 124-126°C (Ethanol).
10) Methansulfonsaeure-(4-acetyl-phenethylamid)
Ausb. 40 % d. Th., Schmp. 80-81°C (Toluol)
11) 3-Chlor-benzolsulfonsaeure-(4-Propionyl-phenethylamid)
Ausb. 96 % d. Th., Schmp. 136-139°C (Toluol)
12) 4-<4-[2-(4-Chlor-benzolsulfonylamino)ethyl]phenyl>-4-oxo-butansaeure-ethylester
Ausb. 90 % d. Th., Schmp. 108-109°C (Ethanol)
13) 4-Chlor-benzolsulfonsaeure-(4-propionyl-benzylamid
Ausb. 89 % d. Th., Schmp. 128°C (Toluol).
14) Benzolsulfonsaeure-(4-propionyl-phenethylamid)
Ausb. 77 % d. Th., Schmp. 116°C (Ethanol).
Oxim: Schmp. 119-120°C
15) 2-Chlor-benzolsulfonsaeure-(4-propionyl-phenylamid)
Ausb. 84 % d. Th., Schmp. 102-103°C (Isopropanol).
16) 4-Chlor-benzolsulfonsaeure-(4-propionyl-phenethylamid)
Ausb. 82 % d. Th., Schmp. 101-102°C (Heptan + Toluol).
17) 4-Brom-benzolsulfonsaeure-(4-propionyl-phenethylamid)
Ausb. 88 % d. Th., Schmp. 99°C (Ethanol)
18) 4-Methoxy-benzolsulfonsaeure-(4-propionyl-phenethylamid)
Ausb. 82 % d. Th., Schmp. 107°C (Isopropanol).
19) 4-Hydroxy-benzolsulfonsaeure-(4-propionyl-phenethylamid)
Ausb. 45 % d. Th., Schmp. 125-126°C (Isopropanol).
20) 4-Cyano-benzolsulfonsaeure-(4-propionyl-phenethylamid)
Ausb. 75 % d. Th., Schmp. 126-127°C (Isopropanol).
21) Benzolsulfonsaeure-(4-butyroyl-phenethylamid)
Ausb. 67 % d. Th., Schmp. 88-90°C (Isopropanol).
22) 4-Chlor-benzolsulfonsaeure-(4-butyroyl-phenethylamid)
Ausb. 82 % d. Th., Schmp. 87-88°C (Toluol + Heptan).
23) 4-Acetylamino-benzolsulfonsaeure-(4-butyroyl)-phenethylamid)
Ausb. 78 % d. Th., Schmp. 141-143°C (Methanol).
24) 4-Chlor-benzolsulfonsaeure-[3-(4-butyroyl-phenyl)propylamid]
Ausb. 85 % d. Th., Schmp. 77-78°C (Toluol)
25) 4-<4-[2-[4-(4-Chlorphenyl)benzolsulfonylamino]ethyl]phenyl>-4-oxo-butansaeure-ethylester
Ausb. 89 % d.Th., Schmp. 157-158°C (Toluol)
26) 4-<4-[2-(Naphthalin-1-sulfonylamino)ethyl]phenyl>-4-oxo-butansaeure-ethylester
Ausb. 71 % d.Th., Schmp. farbloses Oel.
27) 5-<4-[2-(4-Chlor-benzolsulfonylamino)ethyl]phenyl>-5-oxo-pentansaeure-ethylester
Ausb. 97 % d.Th., Schmp. 98-99°C (Essigester)

Das als Ausgangsmaterial verwendete 4-Propionyl-phenethylamin wird auf folgendem Wege dargestellt:

Zu einer Suspension aus 142.5 g (1.55 mol) Propionylchlorid, 96.5 g (0.6 mol) N-Acetyl-phenethylamin und 450 mL 1,1,2,2-Tetrachlorethan gibt man unter Ruehren bei 0-5°C portionsweise 260 g (1.95 mol) $AlCl_3$, ruehrt weitere 5 Stdn. bei Eiskuehlung und gießt schließlich auf Eis. Man trennt nun die org. Phase ab, extrahiert die waeßrige Phase mit Methylenchlorid, vereinigt die Methylenchloridloesung mit der Tetrachlorethan-Loesung und waescht zweimal mit Wasser. Nach Trocknen mittels Natriumsulfat wird i. Vak. eingedampft und der Rueckstand aus einem Essigester-Ligroin-Gemisch umkristallisiert. Ausb. 96.1 g (73 % d. Th.) 4-Propionyl-N-acetyl-phenethylamin mit dem Schmp. 94.5-95°C.

Ein Gemisch aus 60.0 g (273 mmol) 4-Propionyl-N-acetyl-phenethylamin und 500 mL 4 N HCl wird 10 Stdn. im siedenden Wasserbad erhitzt, dann dampft man i. Vak. ein und kristallisiert aus Ethanol um. Ausb. 47.8 g (82 % d. Th.) 4-Propionyl-phenethylamin-hydrochlorid mit dem Schmp. 223-224°C.

Das als Ausgangsmaterial dienende 4-Butyroyl-phenethylamin wird an analoger Weise hergestellt:
4-Butyroyl-N-acetyl-phenethylamin
Ausb. 87 % d. Th., Schmp. 99-101°C (Wasser)
4-Butyroyl-phenethylamin
Ausb. 89 % d. Th., Schmp. d. Hydrochlorids: 228-231°C.

Beispiel 2

4-Brom-benzolsulfonsaeure-[4-(1-hydroxyethyl)phenethylamid]

Man loest 11.4 g (29.8 mmol) 4-Brom-benzolsulfonsaeure-(4-acetyl-phenethylamid) in einem Gemisch aus 40 mL Methanol und 100 mL Ethanol und traegt nun bei 0°C portionsweise 0.85 g (22.4 mmol) Natriumborhydrid ein. Anschließend wird zwei Stunden bei Raumtemperatur geruehrt, dann i. Vak. eingedampft. Nach Zugabe von verd. HCl wird mit Methylenchlorid ausgeschuettelt und die Methylenchloridphase mittels $Na_2SO_4$ getrocknet. Danach dampft man ein und kristallisiert den Rueckstand aus Toluol um.
Ausb. 10.4 g (91% d. Th.), Schmp. 125-127°C.
In Analogie dazu lassen sich herstellen:

2) 5-<4-[2-(4-Chlor-benzolsulfonylamino)ethyl]phenyl>-5-hydroxy-pentansaeure-ethylester
Ausb. 74 % d. Th., Schmp. 89-90°C (Toluol + Ligroin)
aus 5-<4-[2-(4-Chlor-benzolsulfonylamino)ethyl]phenyl>-5-oxo-pentansaeure-ethylester.
3) 4-Chlor-benzolsulfonsaeure-[4-(1-hydroxyethyl)benzylamid]
Ausb. 85 % d. Th., Schmp. 103-104°C (Toluol).
4) Benzolsulfonsaeure-[4-(1-hydroxyethyl)phenethylamid]
Ausb. 64 % d. Th., Schmp. 98-100°C (Toluol).
5) 4-Chlor-benzolsulfonsaeure-[4-1-hydroxyethyl)phenethylamid]
Ausb. 82 % d. Th., Schmp. 105-107°C (Toluol).
6) 3,4-Dichlor-benzolsulfonsaeure-[4-(1-hydroxyethyl)phenethylamid]
Ausb. 96 % d. Th., Schmp. 108-109°C (Heptan + Toluol).
7) 4-Fluor-benzolsulfonsaeure-[4-(1-hydroxyethyl)phenethylamid]
Ausb. 73 % d. Th., Schmp. 99°C (Ethylacetat).
8) 4-Methoxy-benzolsulfonsaeure-[4-(1-hydroxyethyl)phenethylamid]
Ausb. 73 % d. Th., Schmp. 84-85°C (Toluol).

und aus den entsprechenden Benzolsulfonsaeure-(4-propionyl-phenethylamiden) die folgenden Verbindungen:

9) 4-Chlor-benzolsulfonsaeure-[4-(1-hydroxypropyl)benzylamid]
Ausb. 48 % d. Th., Schmp. 102-103°C (Toluol).
10) Benzolsulfonsaeure-[4-(1-hydroxypropyl)phenethylamid]
Ausb. 62 % d. Th., Schmp. 107°C (Essigester).
11) 3-Chlor-benzolsulfonsaeure-[4-(1-hydroxypropyl)phenethylamid]
Ausb. 95 % d. Th., honigartig.
12) 4-Chlor-benzolsulfonsaeure-[4-(1-hydroxypropyl)phenethylamid]
Ausb. 62 % d. Th., Schmp. 135-136°C (Toluol).
13) 4-Brom-benzolsulfonsaeure-[4-(1-hydroxypropyl)phenethylamid]
Ausb. 96 % d. Th., Schmp. 148-149°C (Ethanol).

Aus den entsprechenden Benzolsulfonsaeure-(4-butyroyl-phenethylamiden) lassen in analoger Weise folgende Verbindungen darstellen:

14) Benzolsulfonsaeure-[4-(1-hydroxybutyl) phenethylamid]
Ausb. 73 % d. Th., Schmp. 107-108°C (waeßr. Ethanol).
15) 4-Chlor-benzolsulfonsaeure-[4-(1-hydroxybutyl)phenethylamid]
Ausb. 90 % d. Th., Schmp. 127-128°C (Toluol)
16) 4-Chlor-benzolsulfonsaeure-[3-(4-(1-hydroxybutyl-phenyl)propylamid]
Ausb. 95 % d. Th., Schmp. 78-79°C (Heptan + Toluol).

Beispiel 3

Benzolsulfonsaeure-(4-n-propyl-phenethylamid)

a) 4-n-Propyl-N-acetyl-phenethylamin

Man hydriert in der Schuettelente bei Raumtemperatur und Normaldruck eine methanolische Loesung von 4-Propionyl-N-acetyl-phenethylamin in Gegenwart von Palladium-auf-Kohle-Katalysator, saugt ab, dampft i.Vak. ein und erhaelt in quantitativer Ausbeute 4-n-Propyl-N-acetyl-phenethylamin mit dem Schmp. 52°C.

b) 4-n-Propyl-phenethylamin

Man haelt ein Gemisch aus 9.3 g (45 mmol) 4-n-Propyl-N-acetyl-phenethylamin, 68 mL 2 N NaOH und 40 mL Ethanol 20 h auf Rueckflußtemperatur, kuehlt ab und saeuert mit HCl an. Danach wird das Ethanol abdestilliert und die waeßrige Phase mit Essigester extrahiert. Man macht sie mit KOH stark alkalisch, extrahiert mehrmals mit Ether, trocknet die vereinigten Etherphasen mittels KOH und dampft ein. Es bleiben 4.6 g (62 % d. Th.) oeliges Produkt zurueck, welches ohne weitere Reinigung in die naechste Umsetzung eingebracht wird.

c) Benzolsulfonsaeure-(4-n-propyl-phenethylamid)

Das nach b) erhaltene Produkt wird in Analogie zu Beispiel 1 mit Benzolsulfochlorid umgesetzt, Ausb. 66 % d. Th., farbl. Oel.

Beispiel 4

4-Chlor-benzolsulfonsaeure-[4-(1-propenyl)phenethylamid]

a) 4-Chlor-benzolsulfonsaeure-[4-(1-chlorpropyl)phenethylamid]

Ein Gemisch aus 26.7 g (75.4 mmol) 4-Chlor-benzolsulfonsaeure-[4-(1-hydroxypropyl)phenethylamid, 200 mL Methylenchlorid und 100 mL conc. HCl wird 1 h kraeftig geruehrt, dann verduennt man mit Wasser und trennt die $CH_2Cl_2$-Phase ab.
Man waescht sie mit Wasser und $NaHCO_3$-Loesung, trocknet mit Natriumsulfat und dampft ein. Ausb. quant., Schmp. 90-91°C.

b) 4-Chlor-benzolsulfonsaeure-[4-(1-propenyl)-phenethylamid]

1.86 g (5 mmol) des nach a) erhaltenen Produktes werden im Stickstoffstrom auf 150°C erhitzt, bis die HCl-Entwicklung nachlaeßt. Danach nimmt man in Methylenchlorid auf, verruehrt mit Kieselgel, saugt ab und dampft ein. Der Rueckstand wird in Toluol geloest und an einer kurzen Kieselgel-Saeule chromatographiert.
Ausbeute: 1.04 g (62 % d. Th.), Schmp. 149-150°C.
In analoger Weise stellt man dar:

2) a) Benzolsulfonsaeure-[4-(1-chlorpropyl)phenethylamid]
Ausb. 96 % d. Th., Schmp. 56-57°C
und daraus
b) Benzolsulfonsaeure-[4-(1-propenyl)phenethylamid]
Ausb. 65 % d. Th., Schmp. 40-43°C.
sowie
3) a) 4-Brom-benzolsulfonsaeure-[4-(1-chlorpropyl)phenethylamid]
Ausb. 96 % d. Th., Schmp. 83-85°C.
und daraus
b) 4-Brom-benzolsulfonsaeure-[4-(1-propenyl)phenethylamid]
Ausb. 34 % d. Th., Schmp. 153-155°C.

Beispiel 5

4-Chlor-benzolsulfonsaeure-(4-formyl-phenethylamid)

Ein Gemisch aus 11.0 g (33.8 mmol) 4-Chlor-benzolsulfonsaeure-(4-hydroxymethyl-phenethylamid), 250 mL Methylenchlorid und 30g aktivem Mangandioxid wird 5 Stdn. bei Raumtemperatur geruehrt, dann saugt man das Mangandioxid ab und dampft das Filtrat ein. Ausb. 10.3 g (94 % d. Th.), Schmp. 127-128°C.
Eine andere Oxidationsmethode wurde angewandt zur Herstellung von
4-Chlor-benzolsulfonsaeure-[4-(3-oxopropyl-phenethylamid]
In eine Loesung aus 12.8 g (59.4 mmol) Pyridiniumchlorochromat und 250 mL Methylenchlorid traegt man unter Ruehren bei Raumtemperatur 14.0 g (39.6 mmol) 4-Chlor-benzolsulfonsaeure-[4-(3-hydroxypropyl)phenethylamid] ein. ruehrt eine weitere Stunde und gießt von Ungeloestem ab. Die Methylenchloridphase chromatographiert man mit einer kurzen Saeule (Kieselgel/Methylenchlorid) und dampft ein.
Ausb. 10.8 g (78 % d. Th.), Schmp. 65-66°C.
Oxim: Schmp. 156-158°C (Toluol).

Beispiel 6

4-Chlor-benzolsulfonsaeure-[4-(3-oxo-but-1-enyl)phenethylamid]

Man haelt ein Gemisch aus 3.2 g (10 mmol) 4-Chlor-benzolsulfonsaeure-(4-formyl-phenethylamid], 50 mL abs. Toluol und 3.2 g (10 mmol) 1-Triphenyl-phosphoranyliden-2-propanon 5 Stdn. auf Rueckflußtemperatur, kuehlt dann ab, verruehrt mit Kieselgel und saugt ab. Das Filtrat wird eingedampft und durch Saeulenchlromatografie (Kieselgel/Methylenchlorid) gereinigt.
Ausb. 1.3 g (36 % d. Th.), Schmp. 111-113°C.

Beispiel 7

3-Trifluormethyl-benzolsulfonsaeure-[4-(2-hydroxyethyl)phenethylamid]

a) Durch Umsetzen von 3-Trifluormethyl-benzolsulfochlorid mit 4-(2-Aminoethyl)phenylessigsaeure-ethylester in Analogie zu Beispiel 1 erhaelt man in 76 % d. Th. 4-[2-(3-Trifluormethyl-benzolsulfonylamino)ethyl]phenylessigsaeure-ethylester mit dem Schmp. 95-97°C (Ethylacetat). Die daraus durch Hydrolyse dargestellte Saeure schmilzt bei 119-120°C (Toluol).

b) In eine Suspension aus 0.92 g (24 mmol) Lithiumalanat und 300 mL abs. Ether tropft man bei Raumtemperatur eine Loesung aus 10.0 g (24 mmol) 4-[2-(3-Trifluormethyl-benzolsulfonylamino)ethyl]-phenylessigsaeure-ethylester und 200 mL Ether und haelt anschließend drei Stunden auf Rueckflußtemperatur. Nach dem Zersetzen des Ansatzes mit eiskalter verd. Essigsaeure trennt man die Etherphase ab, schuettelt die waeßrige Phase mit Ether aus, vereinigt die Etherphasen und trocknet mit Natriumsulfat. Man dampft nun ein und kristallisiert den festen Rueckstand aus Toluol um.
Ausb. 7.6 g (85 % d. Th.) der Titelverbindung, Schmp. 70-72°C.

Reduziert man in analoger Weise die entsprechenden Benzoesaeure-ethylester, so erhaelt man zum Beispiel

2) 4-Chlor-benzolsulfonsaeure-(4-hydroxymethyl)-phenethylamid)
Ausb. 85 % d. Th., Schmp. 142-143°C (Toluol + Ethanol).

3) 4-Chlor-benzolsulfonsaeure-[4-(5-hydroxypentyl)-phenethylamid]
Ausb. 59 % d.Th., Schmp. 80-82°C
aus 5-<4-[2-(4-Chlor-benzolsulfonylamino)ethyl]phenyl>-valeriansaeure-ethylester, welcher in Analogie zu Beispiel 1 aus 4-Chlor-benzolsulfochlorid und 5-[4-(2-Aminoethyl )phenyl]valeriansaeure-ethylester dargestellt wird.
Ausb. 89 % d.Th., farbl. Oel.

4) 4-Chlor-benzolsulfonsaeure-[4-(2-hydroxyethyl)benzylamid]
Ausb. 61 % d. Th., Schmp. 125-127°C, (Toluol + Ethanol).
aus 4-(4-Chlor-benzolsulfonylaminomethyl)phenylessigsaeure-ethylester, welcher in Analogie zu Bsp. 1 aus 4-Chlor-benzolsulfochlorid und 4-Aminomethyl-phenylessigsaeure-ethylester dargestellt wird.
Ausb. 81 % d. Th., Schmp. 95-96°C (Toluol).

5) Benzolsulfonsaeure-[4-(2-hydroxyethyl)phenethylamid]
Ausb. 95 % d. Th., Schmp. 70-72°C
aus 4-[2-(Benzolsulfonylamino)ethyl]phenylessigsaeureethylester, welcher in Analogie zu Beispiel 1 aus Benzolsulfochlorid und
4-(2-Aminoethyl)phenylessigsaeure-ethylester dargestellt wird.
Ausb. 87 % d. Th., farbloses Oel.

6) 4-Chlor-benzolsulfonsaeure-[4-(2-hydroxyethyl)phenethylamid]
Ausb. 78 % d. Th., Schmp. 84-86°C (Toluol).
aus 4-[2-(4-Chlor-benzolsulfonylamino)ethyl]phenylessigsaeure-ethylester, welcher in Analogie zu Beispiel 1 aus 4-Chlor-benzolsulfochlorid und 4-(2-Aminoethyl)phenylessigsaeure-ethylester dargestellt wird. Ausb. 84 % d. Th. Schmp. 93-95°C (Heptan + Toluol).

7) 4-Fluor-benzolsulfonsaeure-[4-(2-hydroxyethyl)phenethylamid]
Ausb. 81 % d. Th., Schmp. 78-80°C (Toluol).
aus 4-[2-(4-Fluor-benzolsulfonylamino)ethyl]phenylessigsaeure-ethylester, welcher in Analogie zu Beispiel 1 aus 4-Fluor-benzolsulfochlorid und 4-(2-Aminoethyl)phenylessigsaeure-ethylester dargestellt wird.
Ausb. 87 % d. Th., Schmp. 79-81°C (Ethylacetat).

8) 4-Methoxy-benzolsulfonsaeure-[4-(2-hydroxyethyl)phenethylamid]
Ausb. 58 % d. Th., Schmp. 87-89°C (Toluol).
aus 4-[2-(4-Methoxy-benzolsulfonylamino)ethyl]phenylessigsaeure-ethylester, welcher in Analogie zu Beispiel 1 aus 4-Methoxy-benzolsulfochlorid und 4-(2-Aminoethyl)phenylessigsaeure-ethylester dargestellt wird.
Ausb. 87 % d. Th., Schmp. 85-87°C.

9) 4-Isopropyl-benzolsulfonsaeure-[4-(2-hydroxyethyl)phenethylamid]
Ausb. 92 % d. Th., Schmp. 81-82°C (Toluol).
aus 4-[2-(4-Isopropyl-benzolsulfonylamino)ethyl)phenylessigsaeure-ethylester, welcher in Analogie zu Beispiel 1 aus 4-Isopropyl-benzolsulfochlorid und 4-(2-Aminoethyl)phenylessigsaeure-ethylester dargestellt wird. Ausb. 72 % d. Th., Schmp. 65-67°C (Isopropanol).

Aus den entsprechenden 3-Phenyl-propionsaeureestern lassen sich in analoger Weise zum Beispiel folgende Verbindungen herstellen:

10) 4-Chlor-benzolsulfonsaeure-[4-(5-hydroxypentyl)phenethylamid]
Ausb. 59 % d.Th., Schmp. 80-82°C.
aus 5-<4-[2-(4-Chlor-benzolsulfonylamino)ethyl]phenyl>valeriansaeure-ethylester, welcher in Analogie zu Beispiel 1 aus 4-Chlor-benzolsulfochlorid und 5-[4-(2-Aminoethyl )phenyl]valeriansaeure-ethylester dargestellt wird.
Ausb. 89 % d.Th., farbl. Oel.
11) 4-Chlor-benzolsulfonsaeure-[4-(3-hydroxypropyl)benzylamid]
Ausb. 74 % d. Th., Schmp. 92-94°C (Toluol).
aus 3-[4-(4-Chlor-benzolsulfonylaminomethyl)phenyl]propionsaeure-ethylester, welcher in Analogie zu Beispiel 1 aus 4-Chlor-benzolsulfonylchlorid und
3-[4-Aminomethyl)phenyl]propionsaeure-ethylester dagestellt wird. Ausb. 71 % d. Th., Schmp. 93-94°C (Toluol).
12) 4-Fluor-benzolsulfonsaeure-[4-(3-hydroxypropyl)phenethylamid]
Ausb. 90 % d. Th., Schmp. 77-79°C (Toluol).
aus 3-<4-[2-(4-Fluor-benzolsulfonylamino)ethyl]phenyl>propionsaeure-ethylester, welcher in Analogie zu Beispiel 1 aus 4-Fluor-benzolsulfochlorid und 3-[4-(2-Aminoethyl)phenyl]propionsaeure-ethylester dargestellt wird. Ausb. 85 % d. Th., Schmp. 52-54°C.
(Cyclohexan + Toluol)
13) 4-Chlor-benzolsulfonsaeure-[4-(3-hydroxypropyl)phenethylamid]
Ausb. 75 % d. Th., Schmp. 85-86°C (Heptan + Butylacetat) aus 3-<4-[2-(4-Chlor-benzolsulfonylamino)-ethyl]-phenyl>propionsaeure-ethylester, Schmp. 65°C (Heptan + Toluol).
14) 3-Chlor-benzolsulfonsaeure-[4-(3-hydroxypropyl)phenethylamid]
Ausb. 74 % d. Th., Schmp. 84-86°C (Heptan + Toluol).
aus 3-<4-[2-(3-Chlor-benzolsulfonylamino)ethyl]phenyl>-propionsaeure-ethylester, welcher in Analogie zu Beispiel 1 aus 3-Chlorbenzolsulfochlorid und 3-[4-(2-Aminoethyl)phenyl]propionsaeure-ethylester dargestellt wird.
Ausb. 94 % d. Th., Schmp. 55-57°C.
15) 3-Trifluormethyl-benzolsulfonsaeure-[4-(3-hydroxypropyl)phenethylamid]
Ausb. 74 % d. Th., Schmp. 64-65°C (Ligroin + Toluol).
aus 3-<4-[2-(3-Trifluormethyl-benzolsulfonylamino)ethyl]phenyl>propionsaeure-ethylester, Schmp. 40-41°C (Ligroin).
16) Methansulfonsaeure-[4-(3-hydroxypropyl)phenethylamid]
Ausb. 67 % d. Th., Schmp. 81-81°C (Wasser)
aus 3-<4-[2-(Methansulfonylamino)ethyl]phenyl>propionsaeure-ethylester, welcher in Analogie zu Beispiel 1 aus Methansulfochlorid und 3-[4-(2-Aminoethyl)phenyl]propionsaeure-ethylester dargestellt wird.
Ausb. 78 % d. Th., Schmp. 55-56°C (Ether).
17) n-Hexansulfonsaeure-[4-(3-hydroxypropyl)phenethylamid]
Ausb. 69 % d. Th., Schmp. 83-84°C (Wasser)
aus 3-<4-[2-(n-Hexansulfonsulfonylamino)ethyl]phenyl>propionsaeure-ethylester, welcher in Analogie zu Beispiel 1 aus n-Hexansulfonylchlorid und
3-[4-(2-Aminoethyl)phenyl]propionsaeure-ethylester dargestellt wird.
Ausb. 84 % d. Th., Schmp. 60-61°C (Wasser).
18) Cyclohexansulfonsaeure-[4-(3-hydroxypropyl)phenethylamid]
Ausb. 87 % d. Th.,Schmp. 85-86°C (Toluol)
aus 3-<4-[2-(Cyclohexansulfonylamino)ethyl]phenyl>propionsaeure-ethylester, welcher in Analogie zu Beispiel 1 aus Cyclohexan-sulfonylchlorid und 3-[4-(2-Aminoethyl)phenyl]propionsaeure-ethylester dargestellt wird.
Ausb. 73 % d. Th., farbloses Oel.

Aus entsprechenden 4-Phenyl-buttersaeure-ethylestern werden analog die folgenden (4-Hydroxybutyl)phenyl-Verbindungen dargestellt.

19) 4-Fluor-benzolsulfonsaeure-[4-(4-hydroxybutyl)phenethylamid]
Ausb. 75 % d. Th., Schmp. 82-84°C (Toluol).

aus 4-<4-[2-(4-Fluor-benzolsulfonylamino)ethyl]phenyl>buttersaeure-ethylester,welcher in Analogie zu Beispiel 1 aus 4-Fluor-benzolsulfochlorid und 4-[4-(2-Aminoethyl)phenyl]buttersaeure-ethylester dargestellt wird.
Ausb. 92 % d. Th., farbloses Oel.

20) 3-Chlor-benzolsulfonsaeure-[4-(4-hydroxybutyl)phenethylamid]
Ausb. 84 % d. Th., Schmp. 85-86°C (Heptan + Toluol).
aus 4-<4-[2-(3-Chlor-benzolsulfonylamino)ethyl]phenyl>buttersaeure-ethylester, welcher in Analogie zu Beispiel 1 aus 3-Chlor-benzolsulfochlorid und 4-[4-(2-Aminoethyl)phenyl)buttersaeure-ethylester dargestellt wird.
Ausb. 92 % d. Th., Schmp. 34-36°C (wachsartig).

21) 4-Chlor-benzolsulfonsaeure-[4-(4-hydroxybutyl)phenethylamid]
Ausb. 87 % d. Th., Schmp. 83-85°C (Heptan + Toluol).
aus 4-<4-[2-(4-Chlor-benzolsulfonylamino)ethyl]phenyl>buttersaeure-ethylester, welcher in Analogie zu Beispiel 1 aus 4-Chlor-benzolsulfochlorid und 4-[4-(2-Aminoethyl)phenyl]buttersaeure-ethylester dargestellt wird.
Ausb. 91 % d. Th., Schmp. 55-58°C.

22) 3,4-Dichlor-benzolsulfonsaeure-[4-(4-hydroxybutyl)phenethylamid]
Ausb. 71 % d. Th., Schmp. 95-97°C (Heptan + Toluol).
aus 4-<4-[2-(3,4-Dichlor-benzolsulfonylamino)ethyl]phenyl>buttersaeure-ethylester, welcher in Analogie zu Beispiel 1 aus 3,4-Dichlor-benzolsulfochlorid und 4-[4-(2-Aminoethyl)phenyl]buttersaeure-ethylester dargestellt wird.
Ausb. 86 % d. Th., Schmp. 70-73°C.

23) 4-Brombenzolsulfonsaeure-[4-(4-hydroxybutyl)phenethylamid]
Ausb. 89 % d. Th., Schmp. 84-85°C (Heptan + Toluol).
aus 4-<4-[2-(4-Brom-benzolsulfonylamino)ethyl]phenyl>buttersaeure-ethylester, welcher in Analogie zu Beispiel 1 aus 4-Brom-benzolsulfochlorid und 4-[4-(2-Aminoethyl)phenyl]buttersaeure-ethylester dargestellt wird.
Ausb. 90 % d. Th., Schmp. 63-64°C.

24) 4-Hydroxy-benzolsulfonsaeure-[4-(4-hydroxybutyl)phenethylamid]
Ausb. 63 % d. Th., Schmp. 111-112°C (Toluol).
aus 4-<4-[2-(4-Hydroxy-benzolsulfonylamino)ethyl]phenyl>buttersaeure-ethylester, welcher in Analogie zu Beispiel 1 aus 4-Hydroxy-benzolsulfochlorid und 4-[4-(2-Aminoethyl)phenyl]buttersaeure-ethylester dargestellt wird.
Ausb. 86 % d. Th., farbloses Oel.

25) 4-Chlor-benzolsulfonsaeure-<3-[4-(4-hydroxybutyl)phenyl]propylamid>
Ausb. 81 % d. Th., Schmp. 95-97°C (Toluol).
aus 4-<4-[3-(4-Chlor-benzolsulfonylamino)propyl]phenyl>buttersaeure-ethylester, welcher in Analogie zu Beispiel 1 aus 4-Chlor-benzolsulfochlorid und 4-[4-(3-Aminopropyl)phenyl]buttersaeure-ethylester dargestellt wird.
Ausb. 92 % d. Th., Schmp. 71-73°C (Heptan + Toluol).

26) 3-Trifluormethyl-benzolsulfonsaeure-[4-(4-hydroxybutyl)phenethylamid]
Ausb. 97 % d. Th., Schmp. 72-73°C (Ligroin + Toluol).
aus 4-<4-[2-(3-Trifluormethyl-benzolsulfonylamino)ethyl]phenyl>buttersaeure-ethylester, welcher in Analogie zu Beispiel 1 aus 3-Trifluormethyl-benzolsulfochlorid und 4-[4-(2-Aminoethyl)phenyl]buttersaeure-ethylester dargestellt wird.
Ausb. 92 % d. Th., farbloses Oel.

27) 3-Methoxy-benzolsulfonsaeure-[4-(4-hydroxybutyl)phenethylamid]
Ausb. 77 % d. Th., farbloses Oel, $n_D^{20}$ = 1.5591.
aus 4-<4-[2-(3-Methoxy-benzolsulfonylamino)ethyl]phenyl>buttersaeure-ethylester, welcher in Analogie zu Beispiel 1 aus 3-Methoxy-benzolsulfochlorid und 4-[4-(2-Aminoethyl)phenyl]buttersaeure-ethylester dargestellt wird.
Ausb. 92 % d. Th., farbl. Oel.

28) 3,4-Dimethoxy-benzolsulfonsaeure-[4-(4-hydroxybutyl)phenethylamid]
Ausb. 79 % d. Th., Schmp. 107-109°C (Ethylacetat).
aus 4-<4-[2-(3,4-Dimethoxy-benzolsulfonylamino)ethyl]phenyl>buttersaeure-ethylester, welcher in Analogie zu Beispiel 1 aus 3,4-Dimethoxy-benzolsulfochlorid und 4-[4-(2-Aminoethyl)phenyl]buttersaeure-ethylester dargestellt wird.
Ausb. 72 % d. Th., farbloses Oel.

29) 2-Methyl-benzolsulfonsaeure-[4-(3-hydroxypropyl)phenethylamid]
Ausb. 82 % d.Th., farbl. Oel.
aus 3-<4-[2-(2-Methyl-benzolsulfonylamino)ethyl]phenyl>propionsaeure-ethylester, welcher in Analogie zu Beispiel 1 aus 2-Methyl-benzolsulfochlorid 3-[4-(2-Aminoethyl)phenyl]propionsaeure-ethylester dargestellt wird.
Ausb. 94 % d.Th., Schmp. 50-51°C (Cyclohexan)

30) 1-Naphthalin-sulfonsaeure-[4-(3-hydroxypropyl)phenethylamid]
Ausb. 78 % d.Th., Schmp. 82-83°C (Toluol)
aus 3-<4-[2-(1-Naphthalinsulfonylamino)ethyl]phenyl>propionsaeure-ethylester, welcher in Analogie zu Beispiel 1 aus 1-Naphthalinsulfochlorid und 3-[4-(2-Aminoethyl)phenyl]propionsaeure-ethylester dargestellt wird.
Ausb. 83 % d.Th., Schmp. 73-74°C
31) 4-Chlor-benzolsulfonsaeure-[4-(4-hydroxybutyl)-N-methylphenethylamid]
Ausb. 53 % d.Th., farbloses Oel.
aus 4-<4-[2-(N-Methyl-4-chlor-benzolsulfonylamino)ethyl]phenyl>buttersaeure-ethylester, welcher in Analogie zu Beispiel 1 aus 4-Chlor-benzolsulfochlorid und 4-<4-[2-(Methylamino)ethyl]phenyl>-buttersaeure-ethylester dargestellt wird.
Ausb. 72 % d.Th., farbl. Oel.
32) 4-Chlor-benzolsulfonsaeure-[4-(4-hydroxybutyl)-N-benzylphenethylamid]
Ausb. 68 % d.Th., Schmp. 71-72°C.
aus 4-<4-[2-(N-Benzyl-4-chlor-benzolsulfonylamino)ethyl]phenyl>buttersaeure-ethylester, welcher in Analogie zu Beispiel 1 aus 4-Chlor-benzolsulfochlorid und 4-<4-[2-(Benzylamino)ethyl]phenyl>buttersaeure-ethylester dargestellt wird.
Ausb. 82 % d.Th., Schmp. 70-71°C.

Beispiel 8

4-Azido-benzolsulfonsaeure-[4-(2-hydroxyethyl)phenethylamid]

Zu einer eiskalten Loesung aus 5.5 g (33 mmol) 4-(2-Hydroxyethyl)phenethylamin, 100 mL Methylenchlorid und 3.4 g (33 mmol) Triethylamin tropft man langsam eine Loesung aus 7.2 g (33 mmol) 4-Azidobenzolsulfonylchlorid und 50 mL Methylenchlorid. Anschließend laeßt man eine Stunde bei Eistemperatur nachreagieren. Das Gemisch wird nun zweimal mit verd. Schwefelsaeure, dann zweimal mit Wasser ausgeschuettelt, mit Natriumsulfat getrocknet und schließlich eingedampft. Man kristallisiert zweimal aus Toluol um und erhaelt 7.8 g (64 % d. Th.) Produkt mit dem Schmp. 90-92°C (Toluol).
In Analogie dazu erhaelt man:

2) 4-Cyan-benzolsulfonsaeure-[4-(2-hydroxyethyl)phenethylamid]
Ausb. 51 % d. Th., Schmp. 106-108°C (Toluol)
aus 4-Cyan-benzolsulfochlorid und 4-(2-Hydroxyethyl)phenethylamin.
3) 4-Brom-benzolsulfonsaeure-[4-(3-hydroxypropyl)phenethylamid]
Ausb. 78 % d. Th., Schmp. 82-84°C (Heptan + Toluol)
aus 4-Brom-benzolsulfochlorid und 4-(3-Hydroxypropyl)phenethylamin.
4) 4-Azido-benzolsulfonsaeure-[4-(3-hydroxypropyl)phenethylamid]
Ausb. 39 % d. Th., Schmp. 85-88°C (Toluol)
aus 4-Azido-benzolsulfochlorid und 4-(3-Hydroxypropyl)phenethylamin.
5) 4-Nitro-benzolsulfonsaeure-[4-(3-hydroxypropyl)phenethylamid]
Ausb. 69 % d. Th., Schmp. 117-118°C (Ethanol)
aus 4-Nitro-benzolsulfochlorid und 4-(3-Hydroxypropyl)phenethylamin.
6) 4-Cyan-benzolsulfonsaeure-[4-(4-hydroxybutyl)phenethylamid]
Ausb. 55 % d. Th., Schmp. 107-109°C (Toluol)
aus 4-Cyan-benzolsulfochlorid und 4-(4-Hydroxybutyl)phenethylamin.
7) 4-Acetyl-benzolsulfonsaeure-[4-(4-hydroxybutyl)phenethylamid]
Ausb. 56 % d. Th., Schmp. 109°C (Toluol)
aus 4-Acetyl-benzolsulfochlorid und 4-(4-Hydroxybutyl)phenethylamin.
8) 4-Chlor-styrolsulfonsaeure-[4-(3-hydroxypropyl)phenethylamid]
Ausb. 62 % d.Th., Schmp. 111-112°C (Toluol).
aus 4-Chlor-styrolsulfochlorid und 4-(3-Hydroxypropyl)phenethylamin.
9) 4-Chlor-benzolsulfonsaeure-[4-(1,3-dihydroxypropyl)phenethylamid]
Ausb. 23 % d. Th., Schmp. 80-82°C (Wasser
aus 4-Chlor-benzolsulfochlorid und 4-(1,3-Dihydroxypropyl)phenethylamin.

Beispiel 9

4-Chlor-phenylethansulfonsaeure-[4-(3-hydroxypropyl)phenethylamid]

a) Durch Umsetzen von 4-Chlor-phenylethansulfochlorid mit 3-[4-(2-Aminoethyl)phenyl]propionsaeure-ethylester in Analogie zu Beispiel 1 erhaelt man 3-<4-[2-(4-Chlor-phenylethansulfonylamino)-ethyl]phenyl>propionsaeure-ethylester. Ausb. 80 % d. Th., Schmp. 77-78°C (Ethanol).

b) Zu einer siedenden Loesung aus 12.2 g (28 mmol) des nach a) erhaltenen Ethylesters, 112 mL tert. Butanol und 2.99 g (79 mmol) Natriumborhydrid tropft man innerhalb einer Stunde 22.4 mL Methanol, haelt eine weitere Stunde auf Rueckflußtemperatur und kuehlt dann ab.
Zu der sirupoesen Fluessigkeit gibt man Wasser, engt im Vakuum ein und extrahiert nun mit Methylenchlorid. Die Methylenchloridphase wird mit $Na_2SO_4$ getrocknet, dann dampft man ein und kristallisiert den Rueckstand aus Toluol um. Ausb. 9.08 g (85 % d. Th.) Titelverbindung mit dem Schmp. 94-95°C.

Beispiel 10

4-<4-[2-(3-Chlor-benzolsulfonylamino)ethyl]phenyl>-4-oxo-butansaeure

a) 4-<4-[2-(Acetamino)ethyl]phenyl>-4-oxo-butansaeure-ethylester

Zu einer Loesung aus 120.0 g (0.74 mol) N-Acetyl-phenethylamin und 1.4 L 1,1,2,2,-Tetrachlorethan gibt man 146.0 g (0.89 mol) Bernsteinsaeure-ethylesterchlorid. Dann wird abgekuehlt und bei 0°C portionsweise 293 g Aluminiumchlorid zugegeben. Man ruehrt noch 1 h bei 0°C, anschließend 4 h bei Raumtemperatur, gießt in ein Eis-Salzsaeuregemisch und trennt die Tetrachlorethanphase ab. Sie wird mit Wasser und Sodaloesung gewaschen, mit $Na_2SO_4$ getrocknet und eingedampft. Der Rueckstand wird aus Essigester umkristallisiert.
Ausb. 157.4 g (73 % d. Th.), Schmp. 111-112°C.

b) 4-[4-(2-Aminoethyl]phenyl]-4-oxo-butansaeure

Ein Gemisch aus 180.0 g (0.62 mol) des nach a) erhaltenen Produktes und 1.3 L 6 N HCl wird 8 h auf Rueckflußtemp. gehalten, dann kuehlt man in Eisbad und saugt die ausgefallenen Kristalle ab. Nach Waschen mit eiskalter verd. HCl und Trocknen ueber KOH erhaelt man 127.5 g (76 % d. Th.) Hydrochlorid mit dem Schmp. 232-234°C (Zers.).

c) 4-[4-(2-Aminoethyl)phenyl]-4-oxo-butansaeure-ethylester

Auf ein Gemisch aus 127.2 g (0.49 mol) der nach b) erhaltenen Saeure und 800 mL Ethanol leitet man unter Ruehren HCl-Gas und erhitzt 2 h auf Rueckflußtemperatur. Dann wird stark abgekuehlt. Man saugt die ausgefallenen Kristalle ab, waescht sie mit kaltem Ethanol und trocknet sie ueber $CaCl_2$. Ausb. 137.1 g (97 % d. Th.) Hydrochlorid mit dem Schmp. 162-165°C.

d) 4-<4-[2-(3-Chlor-benzolsulfonylamino)ethyl]phenyl>-4-oxo-butansaeure-ethylester

durch Umsetzen des nach c) erhaltenen Produktes mit 3-Chlor-benzolsulfochlorid in Analogie zu Beispiel 1.
Ausb. 81 % d. Th., Schmp. 64-66°C (Heptan + Toluol).

e) 4-<4-[2-(3-Chlor-benzolsulfonylamino)ethyl]phenyl>-4-oxo-butansaeure

Ein Gemisch aus 5.0 g (11.8 mmol) des nach d) erhaltenen Produktes, 18 mL 2 N NaOH und 20 mL Methanol wird 3 h bei 50°C geruehrt, dann destilliert man i. Vak. das Methanol ab und extrahiert die waeßrige Loesung mit Ether. Anschließend saeuert man mit 6 N HCl an, saugt die ausgefallene Saeure ab, waescht sie mit Wasser und trocknet ueber KOH.
Ausb. 4.6 g (quant. Ausb.), Schmp. 126-128°C.
In Analogie dazu erhaelt man durch Umsetzen mit den entsprechenden Sulfonsaeurechloriden und anschließende Verseifung die folgenden Verbindungen:

2) 4-[4-(4-Chlor-benzolsulfonylaminomethyl)phenyl-4-oxo-butansaeure-ethylester
Ausb. 97 % d. Th., Schmp. 104-106°C (Xylol)
und daraus
4-[4-(4-Chlor-benzolsulfonylaminomethyl)phenyl]-4-oxo-butansaeure
Ausb. 76 % d. Th., Schmp. 185-186°C (Ethylacetat).
3) 4-<4-[2-(4-Fluor-benzolsulfonylamino)ethyl]phenyl>-4-oxo-butansaeure-ethylester
Ausb. 76 % d. Th., Schmp. 104-105°C (Ethylacetat)
und daraus
4-<4-[2-(4-Fluor-benzolsulfonylamino)ethyl]phenyl>-4-oxo-butansaeure
Ausb. 75 % d. Th., Schmp. 138°C (Ethylacetat).
4) 4-<4-[2-(4-Brom-benzolsulfonylamino)ethyl]phenyl>-4-oxo-butansaeure-ethylester
Ausb. 77 % d. Th., Schmp. 108-109°C (Toluol + Heptan).
und daraus

4-<4-[2-(4-Brom-benzolsulfonylamino)ethyl]phenyl>-4-oxo-butansaeure
Ausb. 96 % d. Th., Schmp. 145-148°C.

5) 4-<4-[2-(3-Trifluormethyl-benzolsulfonylamino)ethyl]phenyl>-4-oxo-butansaeure-ethylester
Ausb. 86 % d. Th., Schmp. 83-85°C (Toluol)
und daraus
4-<4-[2-(3-Trifluormethyl-benzolsulfonylamino)ethyl]phenyl>-4-oxo-butansaeure
Ausb. 87 % d. Th., Schmp. 120-121°C (Toluol + Ethylacetat).

6) 4-<4-[2-(2-Methoxy-benzolsulfonylamino)ethyl]phenyl>-4-oxo-butansaeure-ethylester
Ausb. 87 % d. Th., Schmp. 82-84°C (Toluol)
und daraus
4-<4-[2-(2-Methoxy-benzolsulfonylamino)ethyl]phenyl>-4-oxo-butansaeure
Ausb. 83 % d. Th., Schmp. 146°C (Ethanol).

7) 4-<4-[2-(4-Chlor-phenethylsulfonylamino)ethyl]phenyl>-4-oxo-butansaeure-ethylester
Ausb. 88 % d. Th., Schmp. 112°C (Ethanol)
und daraus
4-<4-[2-(4-Chlor-phenethylsulfonylamino)ethyl]phenyl>-4-oxo-butansaeure
Ausb. 74 % d. Th., Schmp. 143-144°C (Ethanol).

8) 4-<4-[2-(4-Chlor-styrolsulfonylamino)ethyl]phenyl>-4-oxo-butansaeure-ethylester
Ausb. 83 % d. Th., Schmp. 98-100°C (Ethanol)
und daraus
4-<4-[2-(4-Chlor-styrolsulfonylamino)ethyl]phenyl>-4-oxo-butansaeure
Ausb. 68 % d. Th., Schmp. 152-153°C (Toluol + Ethylacetat).

9) 4-<4-[2-(4-Hydroxy-benzolsulfonylamino)ethyl]phenyl>-4-oxo-butansaeure-ethylester
Ausb. 64 % d. Th., Schmp. 139-142°C (Essigsaeure)
und daraus
4-<4-[2-(4-Hydroxy-benzolsulfonylamino)ethyl]phenyl>-4-oxo-butansaeure
Ausb. 81 % d. Th., Schmp. 163-164°C (Ethanol).

10) 4-<4-[2-(4-Methoxy-benzolsulfonylamino)ethyl]phenyl>-4-oxo-butansaeure-ethylester
Ausb. 85 % d. Th., Schmp. 83-84°C (Toluol)

11) 4-<4-[2-(4-Methyl-benzolsulfonylamino)ethyl]phenyl>-4-oxo-butansaeure-ethylester
Ausb. 71 % d. Th., Schmp. 91-92°C (Toluol)

Unter Verwendung von 4-[4-(3-Aminopropyl)phenyl]-4-oxo-butansaeure-ethylester wird dargestellt:

12) 4-<4-[3-(4-Chlor-benzolsulfonylamino)propyl]phenyl>-4-oxo-butansaeure-ethylester
Ausb. 91 % d. Th., Schmp. 84°C (Toluol)
und daraus
4-<4-[3-(4-Chlor-benzolsulfonylamino)propyl]phenyl>-4-oxo-butansaeure
Ausb. 95 % d. Th., Schmp. 147-149°C.

Beispiel 11

4-<4-[2-(4-Chlor-benzolsulfonylamino)ethyl]phenyl>-4-oxo-butansaeure

Zu einer Loesung aus 7.8 g (30 mmol) 4-[4-(2-Aminoethyl)phenyl]-4-oxo-butansaeure-hydrochlorid, 45 mL Wasser und 8.4 g (60 mmol) Kaliumcarbonat gibt man bei 80°C portionsweise 7.2 g (34 mmol) 4-Chlor-benzolsulfochlorid, ruehrt weitere 1.5 h bei 80°C und kuehlt dann ab. Nun wird angesaeuert, die ausgefallene Saeure abgesaugt, mit Wasser gewaschen und getrocknet. Ausb. 8.3 g (69 % d. Th.), Schmp. 152°C (waeßriges Aceton).

Beispiel 12

4-<4-[2-(4-Brom-benzolsulfonylamino)ethyl]phenyl>-4-hydroxy-butansaeure-ethylester

Zu einem Gemisch aus 8.0 g (17.1 mmol) 4-<4-[2-(4-Brom-benzolsulfonylamino)ethyl]phenyl>-4-oxo-butansaeure-ethylester, 80 mL Ethanol und 30 mL Methanol gibt man unter Ruehren und Eiskuehlung 0.49 g (12.8 mmol) $NaBH_4$ portionsweise zu. Danach wird 2 h bei Raumtemperatur geruehrt, dann i. Vak. eingedampft. Man gibt Methylenchlorid und verd. HCl zu, schuettelt durch und trennt die Phasen. Die $CH_2Cl_2$-Phase wird mit Wasser gewaschen. mit $Na_2SO_4$ getrocknet und eingedampft. Nach Umkristallisieren aus Ethanol 5.4 g (67 % d. Th.), Schmp. 99-100°C.

In analoger Weise erhaelt man folgende Ester:

2) 4-[4-(4-Chlor-benzolsulfonaminomethyl)phenyl]-4-hydroxy-butansaeure-ethylester
Ausb. 66 % d. Th., Schmp. 93-95°C (Ethanol).

3) 4-<4-[2-(4-Fluor-benzolsulfonylamino)ethyl]phenyl>-4-hydroxy-butansaeure-ethylester
Ausb. 71 % d. Th., Schmp. 82-83°C (Ethylacetat + Ligroin).
4) 4-<4-[2-(3-Chlor-benzolsulfonylamino)ethyl]phenyl>-4-hydroxy-butansaeure-ethylester
Ausb. 67 % d. Th., Schmp. 78-81°C (Ethanol).
5) 4-<4-[2-(4-Chlor-benzolsulfonylamino)ethyl]phenyl>-4-hydroxy-butansaeure-ethylester
Ausb. 92 % d. Th., farbl. Oel.
6) 4-<4-[2-(3-Trifluormethyl-benzolsulfonylamino)ethyl]phenyl>-4-hydroxy-butansaeure-ethylester
Ausb. 81 % d. Th., farbl. Oel.
7) 4-<4-[2-(2-Methoxy-benzolsulfonylamino)ethyl]phenyl>-4-hydroxy-butansaeure-ethylester
Ausb. 74 % d. Th., farbl. Oel, $n_D^{20}$ = 1.5417.
8) 4-<4-[2-(4-Chlor-phenethylsulfonylamino)ethyl]phenyl>-4-hydroxy-butansaeure-ethylester
Ausb. 97 % d. Th., farbl. Oel.

Beispiel 13

Lacton der 4-<4-[2-(3-Trifluormethyl-benzolsulfonylamino)ethyl]phenyl>-4-hydroxy-butansaeure.

Zu einer eiskalten Loesung aus 9.0 g (19.6 mmol) 4-<4-[2-(3-Trifluormethyl-benzolsulfonylamino)ethyl]phenyl>-4-oxo-butansaeure-ethylester und 150 mL Ethanol gibt man in kleinen Portionen 1.0g (26.3 mmol) $NaBH_4$ und ruehrt anschließend 4 Stdn. bei Raumtemperatur. Dann wird i. Vak. das Ethanol abdestilliert, der Rueckstand wird mit Eiswasser verduennt und mittels verd. Schwefelsaeure angesaeuert. Es faellt ein Oel aus, welches durchkristallisiert. Man saugt ab, trocknet und kristallisiert aus Toluol um. Ausb. 7.8 g (86 % d. Th.) Lacton, Schmp. 98-100°C.

Beispiel 14

Lacton der 4-<4-[2-(4-Brom-benzolsulfonylamino)ethyl]phenyl>-4-hydroxy-butansaeure

erhaelt man durch 4 Stdn. Erhitzen einer Loesung aus 4.42 g (10 mmol) 4-<4-[2-(4-Brom-benzolsulfonylamino)ethyl]phenyl>-4-hydroxy-butansaeure, 120 mL Toluol und einer Spitze p-Toluolsulfonsaeure. Nach dem Abkuehlen wird mit $NaHCO_3$-Loesung ausgeschuettelt, mit $Na_2SO_4$ getrocknet und i. Vak. eindampft.
Ausb.: quantitativ, Schmp. 95-98°C.
In analoger Weise erhaelt man das
2) Lacton der 4-<4-[2-(3-Chlor-benzolsulfonylamino)ethyl]phenyl>-4-hydroxy-butansaeure
Ausb. quantitativ, Schmp. 78-79°C.

Beispiel 15

4-<4-[2-(4-Chlor-benzolsulfonylamino)ethyl]phenyl>-4-hydroxy-butansaeure

Man haelt ein Gemisch aus 11.0 g (25.8 mmol) Ethylester obiger Saeure, 30 mL Ethanol und 50 mL 2 N NaOH 5 Stdn. auf Rueckflußtemperatur, destilliert das Ethanol ab und verduennt den Rueckstand mit Wasser. Die waeßrige Phase wird mit Ethylacetat extrahiert, dann saeuert man mit 2 N $H_2SO_4$ an und extrahiert wiederum mit Ethylacetat. Die organische Phase wird mit $Na_2SO_4$ getrocknet, dann eingedampft.
Ausb. 7.1 g (69 % d. Th.), Schmp. 94-95°C (Ethylacetat + Ligroin).
In analoger Weise werden dargestellt:

2) 4-<4-[2-(4-Fluor-benzolsulfonylamino)ethyl]phenyl>-4-hydroxy-butansaeure
Ausb. 93 % d. Th., Schmp. 94-95°C.
3) 4-<4-[2-(Benzolsulfonylamino)ethyl]phenyl>-4-oxo-butansaeure
Ausb. 97 % d.Th., Schmp. 155-156°C (Toluol + Essigester)
4) 4-<4-[2-(Naphthalin-1-sulfonylamino)ethyl]phenyl>-4-oxo-butansaeure
Ausb. 85 % d.Th., Schmp. 158-159°C (Ethanol)
5) 4-<4-[2-(N-Methyl-4-chlor-benzolsulfonylamino)ethyl]phenyl>-4-oxo-butansaeure
Ausb. 76 % d.Th., Schmp. 118-119°C (Ethanol)
6) 4-<4-[2-(N-benzyl-4-chlor-benzolsulfonylamino)ethyl]phenyl>-4-oxo-butansaeure
Ausb. 84 % d.Th., Schmp. 157-158°C (Ethanol + Essigester)
7) 4-<4-[2-[N-(4-Chlor-cinnamyl)-4-chlor-benzolsulfonylamino]ethyl]phenyl>-4-oxo-butansaeure
Ausb. 80 % d.Th., Schmp. 125-126°C (Ethanol)
8) 5-<4-[2-(4-Chlor-benzolsulfonylamino)ethyl]phenyl>-5-hydroxy-pentansaeure
Ausb. 91 % d.Th., Schmp. 142-143°C (Essigester)
9) 5-<4-[2-(4-Chlor-benzolsulfonylamino)ethyl]phenyl>-5-oxo-pentansaeure
Ausb. 88 % d.Th., Schmp. 175-176°C (Ethanol)

10) 4-<4-[2-(4-(4-Chlor-phenyl)benzolsulfonylamino)ethyl]phenyl>-4-oxo-butansaeure
Ausb. 88 % d.Th., Schmp. 191°C (Eisessig)

Beispiel 16

4-<4-[2-(4-Brom-benzolsulfonylamino)ethyl]phenyl>-4-hydroxy-butansaeure

Man loest das Lacton dieser Hydroxysaeure in der Waerme in der berechneten Menge 2 N NaOH, kuehlt ab und gibt nun die der Natronlauge aequivalente Menge Essigsaeure zu, wobei die Saeure ausfaellt. Nach Waschen mit Wasser und Trocknen (Raumtemp., ueber KOH) in praktisch quantitativer Ausbeute die Saeure (nach MS und DC bestaetigt), welche bei der Schmelzpunktbestimmung sofort wieder lactonisiert.

Beispiel 17

Dinatriumsalz der 4-<4-[2-(3-Chlor-benzolsulfonylamino)ethyl]phenyl>-4-hydroxy-butansaeure

Man erwaermt ein Gemisch aus dem Lacton der obigen Hydroxy-butansaeure mit exakt zwei Aequivalenten Natronlauge (in Form einer 0.1 N NaOH), dampft i. Vak. ein und trocknet im Vakuumexsikkator ueber KOH.
Ausbeute: quantitativ, Schmp. ab. 145°C.

Beispiel 18

4-Hydroxy-benzolsulfonsaeure-[4-(1,4-dihydroxybutyl)phenethylamid]

Zu einer Suspension aus 1.1 g (29 mmol) $LiAlH_4$ und 50 mL abs. Tetrahydrofuran tropft man bei Raumtemperatur eine Loesung aus 5.9 g (14.5 mmol) 4-<4-[2-(4-Hydroxy-benzolsulfonylamino)ethyl]-phenyl>-4-oxo-butansaeure-ethylester und 100 mL abs. Tetrahydrofuran und haelt anschließend 6 Stdn. auf Rueckflußtemperatur. Dann wird mit waeßriger Essigsaeure zersetzt, vom Hydroxid-Niederschlag abgesaugt und eingedampft. Man nimmt den Rueckstand in Ethylacetat auf, trocknet mit $Na_2SO_4$, dampft ein und kristallisiert aus Ethylacetat um.
Ausb. 2.6 g (61 % d. Th.), Schmp. 133-135°C.
In analoger Weise werden dargestellt:

2) 4-Chlor-benzolsulfonsaeure-[4-(1,4-dihydroxybutyl)benzylamid]
Ausb. 56 % d. Th., Schmp. 142-143°C (Ethanol).
3) 4-Fluor-benzolsulfonsaeure-[4-(1,4-dihydroxybutyl)phenethylamid]
Ausb. 62 % d. Th., Schmp. 75-77°C (Ligroin + Ether).
4) 3-Chlor-benzolsulfonsaeure-[4-(1,4-dihydroxybutyl)phenethylamid]
Ausb. 71 % d. Th., Schmp. 75-76°C (Butylacetat).
5) 4-Chlor-benzolsulfonsaeure-[4-(1,4-dihydroxybutyl)phenethylamid]
Ausb. 55 % d. Th., Schmp. 101°C.
6) 2-Methoxy-benzolsulfonsaeure-[4-(1,4-dihydroxybutyl)phenethylamid]
Ausb. 74 % d. Th., farbl. Oel; $n_D^{20} = 1.5571$.
7) 4-Methyl-benzolsulfonsaeure-[4-(1,4-dihydroxybutyl)phenethylamid]
Ausb. 79 % d. Th., Schmp. 103-105°C (Ethylacetat).
8) 4-Methoxy-benzolsulfonsaeure-[4-(1,4-dihydroxybutyl)phenethylamid]
Ausb. 65 % d. Th., Schmp. 78-80°C (Ethylacetat).
9) 4-Chlor-benzolsulfonsaeure-<3-[4-(1,4-dihydroxybutyl)phenyl]propylamid>
Ausb. 85 % d. Th., Schmp. 110-112°C (Heptan + Toluol).
10) 3-Trifluormethyl-benzolsulfonsaeure-[4-(1,4-dihydroxybutyl)phenethylamid]
Ausb. 96 % d.Th., farbl. Oel.
11) Benzolsulfonsaeure-[4-(1,4-dihydroxybutyl)phenethylamid]
Ausb. 71 % d.Th., Schmp. 96-97°C (Toluol)

Beispiel 19

4-Brom-benzolsulfonsaeure-[4-(1,4-dihydroxybutyl)phenethylamid]

Zu einer Loesung aus 4.0 g (8.5 mmol) 4-<4-[2-(4-Brom-benzolsulfonylamino)ethyl]phenyl>-4-hydroxy-butansaeure-ethylester und 40 mL tert. Butanol gibt man. 1.0 g (25.5 mmol) $NaBH_4$, erhitzt nun auf Rueckflußtemperatur und tropft innerhalb 1 Std. 6.8 mL Methanol zu. Nach einer weiteren Stunde Erhitzen wird eingedampft, mit 2 N $H_2SO_4$ angesaeuert und mit Methylenchlorid ausgeschuettelt. Man trock-

net die $CH_2Cl_2$-Phase mit $Na_2SO_4$, dampft ein und kristallisiert aus Butylacetat um. Ausb. 3.36 g (82 % d. Th.), Schmp. 125-127°C.

In analoger Weise wird dargestellt:

2) 4-Chlor-phenethylsulfonsaeure-[4-(1,4-dihydroxybutyl)phenethylamid]
Ausb. 76 % d. Th., Schmp. 105-106°C (Toluol).
aus 4-<4-[2-(4-Chlor-phenethylsulfonylamino)ethyl]phenyl>-4-hydroxy-butansaeure-ethylester

3) 4-Chlor-benzolsulfonsaeure-[4-(1,5-dihydroxypentyl)phenethylamid]
Ausb. 76 % d.Th., Schmp. 107-108°C
aus 5-<4-[2-(4-Chlor-benzolsulfonylamino)ethyl]phenyl>5-hydroxy-pentansaeure-ethylester

Beispiel 20

Benzolsulfonsaeure-[4-(4-hydroxy-1-oxo-butyl)phenethylamid

Zu einer Loesung aus 6.0 g (17.2 mmol) Benzolsulfonsaeure-[4-(1,4-dihydroxybutyl)phenethylamid] und 100 mL Methylenchlorid gibt man unter Ruehren langsam 30 g Mangandioxid, ruehrt ca. 15 min nach und saugt ab. Nach Eindampfen und Umkristallisieren aus Essigsaeure-butylester, danach aus einem Toluol-Ethanol-Gemisch erhaelt man 3.2 g (54 % d.Th.) Produkt mit dem Schmp. 89-90°C. Beim Erwaermen tritt leicht Zersetzung ein.

Beispiel 21

4-Chlor-benzolsulfonsaeure-[4-(2-acetoxyethyl)-N-acetyl-phenethylamid]

Zu einer Suspension aus 5.5 g (16 mmol) 4-Chlor-benzolsulfonsaeure-[4-(2-hydroxyethyl)phenethylamid], 100 mL Methylenchlorid und 3.6 g (36 mmol) Triethylamin tropft man bei 0°C langsam eine Loesung aus 2.54 g (32 mmol) Acetylchlorid und 25 mL Methylenchlorid und ruehrt dann zwei Stdn. bei Raumtemperatur nach. Anschließend wird mit Natriumcarbonatloesung, mit verd. Salzsaeure und mehrmals mit Wasser gewaschen, mit Natriumsulfat getrocknet und eingedampft. Nach Chromatografie an Kieselgel/Methylenchlorid 4.0 g (56 % d.Th.) farbloses Oel.

In analoger Weise wird dargestellt
Benzolsulfonsaeure-[4-(1-benzoyloxy-propyl)-N-benzoylphenethylamid]
Ausb. 82 % d.Th., farbl. Oel
aus Benzolsulfonsaeure-[4-(1-hydroxypropyl)phenethylamid] und 2 mol Benzoylchlorid.

Beispiel 22

4-Chlor-benzolsulfonsaeure-(4-propionyl-N-acetyl-phenethylamid]

Zu einem eiskalten Gemisch aus 16.0 g (45.5 mmol) 4-Chlor-benzolsulfonsaeure-(4-propionyl-phenethylamid), 5.1 mL (50 mmol) Triethylamin und 200 mL Methylenchlorid tropft man langsam eine Loesung aus 3.57 g (45.5 mmol) Acetylchlorid und 50 mL Methylenchlorid. Man ruehrt drei Stdn. bei Raumtemp. nach, extrahiert mit 2 N-HCl und mehrmals mit Wasser, trocknet mit $Na_2SO_4$ und dampft ein. Nach Chromatografie an Kieselgel/Toluol + Essigester (12:1 Vol) erhaelt man 10.2 g (57 % d.Th.) Produkt mit dem Schmp. 91-92°C (Heptan + Toluol).

In analoger Weise werden dargestellt:
Aus 4-<4-[2-(4-Chlor-benzolsulfonylamino)ethyl]phenyl>-4-oxo-butansaeure-ethylester

2) und Acetylchlorid:
4-<4-[2-(N-Acetyl-4-chlor-benzolsulfonylamino)ethyl]phenyl>-4-oxo-butansaeure-ethylester
Ausb. 64 % d.Th., Schmp. 107-108°C

3) und Octanoylchlorid:
4-<4-[2-(N-Octanoyl-4-chlor-benzolsulfonylamino)ethyl]phenyl>-4-oxo-butansaeure-ethylester
Ausb. 69 % d.Th., Schmp. 85-86°C (Ethanol).

4) und Palmitoylchlorid:
4-<4-[2-(N-Palmitoyl-4-chlor-benzolsulfonylamino)ethyl]phenyl>-4-oxo-butansaeure-ethylester
Ausb. 65 % d.Th., Schmp. 86-87°C (Ethanol)

5) und 4-Chlor-benzoylchlorid:
4-<4-[2-(N-(4-Chlor-benzoyl)-4-chlor-benzolsulfonylamino)ethyl]phenyl>-4-oxo-butansaeure-ethylester
Ausb. 67 % d.Th., Schmp. 104-105°C (Ethanol).

Beispiel 23

4-Chlor-benzolsulfonsaeure-[4-(2-hydroxyethyl)-N-octanoyl-phenethylamid]

Zu einem Gemisch aus 5.5 g (16 mmol) 4-Chlor-benzolsulfonsaeure-[4-(2-hydroxyethyl)phenethylamid], 1.8 g (18 mmol) Triethylamin und 100 mL Methylenchlorid tropft man bei 0°C langsam eine Loesung aus 2.63 g (16 mmol) Octanoylchlorid und 25 mL Methylenchlorid. Dann haelt man eine Std. bei 0°C und weitere zwei Stdn. bei Raumtemperatur, schuettelt anschließend mit verd. Salzsaeure und Wasser aus, trocknet mit $Na_2SO_4$ und dampft ein. Nach Chromatografie (Kieselgel/Methylenchlorid) 3.8 g (51 % d.Th.) mit dem Schmp. 55°C.

In analoger Weise erhaelt man

2) mit 4-Chlor-benzoylchlorid die Verbindung
4-Chlor-benzolsulfonsaeure-[4-(2-hydroxyethyl)-N-(4-chlor-benzoyl)phenethylamid
Ausb. 59 % d.Th., Schmp. 112°C.

Beispiel 24

4-Chlor-benzolsulfonsaeure-[4-(4-hydroxybutyl)-N-(4-chlor-cinnamyl)phenethylamid]

a) 4-<4-[2-[N-(4-Chlor-cinnamyl)-4-chlor-benzolsulfonylamino]ethyl]phenyl>buttersaeure-ethylester KS 1749

Zu einer Loesung aus 10.0 g (24.4 mmol) 4-<4-[2-(4-Chlor-benzolsulfonylamino)ethyl]phenyl>buttersaeure-ethylester und 80 mL Ethanol gibt man eine Natriummethylatloesung, die 24.4 mg-Atom Natrium enthaelt, dampft vollstaendig ein, verruehrt mit Ether und saugt unter Feuchtigkeitsausschluß ab. Man loest das Salz in 50 mL abs. DMF, gibt. 4.62 g (24.7 mmol) 4-Chlor-cinnamylchlorid zu und haelt nun 3 Stdn. bei 80°C. Nach dem Abkuehlen wird in Wasser eingeruehrt, mit Ether ausgeschuettelt und die Etherphase mittels Natriumsulfat getrocknet. Nach dem Eindampfen erhaelt man 11.9 g (87 % d.Th.) Produkt mit dem Schmp. 65-67°C.

b) 4-<4-[2-[N-(4-Chlor-cinnamyl)-4-chlor-benzolsulfonylamino]ethyl]phenyl>buttersaeure

durch Verseifen des nach a) erhaltenen Esters in Analogie zu Beispiel 15.
Ausb. 78 % d.Th., Schmp. 112-113°C (Ethanol)

c) Titelverbindung

Zu einem Gemisch aus 4.5 g (8.5 mmol) der nach b) erhaltenen Saeure, 0.86 g (8.5 mmol) abs. Triethylamin und 30 mL abs. THF tropft man innerhalb 15 min bei -5°C eine Loesung aus 0.92 g (8.5 mmol) Chlorameisensaeure-ethylester und 15 mL abs. THF. Dann wird 30 min bei -5°C nachgeruehrt, das ausgefallene Triethylammoniumchlorid abgesaugt und das Filtrat innerhalb von 30 min bei 10-15°C zu einer waessr. Suspension von 0.8 g (21 mmol) Natriumborhydrid getropft. Nach zwei Stdn. Ruehren bei Raumtemperatur wird THF abdestilliert, der Rueckstand in 2 N-NaOh geloest und die NaOH-Phase mehrmals mit $CH_2Cl_2$ extrahiert. Man trocknet mit $MgSO_4$, dampft ein und erhaelt nach Chromatografie (Kieselgel/$CH_2Cl_2$) 2.4 g (55 % d.Th.) Produkt mit dem Schmp. 80°C.

In aehnlicher Weise erfolgt die Darstellung von

2) 4-Chlor-benzolsulfonsaeure-[4-(2-hydroxyethyl)-N-methyl-phenethylamid]
Ausb. 61 % d.Th., farbloses Oel,
indem man 2-<4-[2-(4-Chlor-benzolsulfonylamino)ethyl]phenyl>essigsaeure-ethylester mit Methyljodid umsetzt und den resultierenden 2-<4-[2-(N-Methyl-4-chlor-benzolsulfonylamino)ethyl]phenyl>essigsaeure-ethylester (Ausb. 56 % d.Th., farbloses Oel) mit $LiAlH_4$ reduziert.

Beispiel 25

Unter Anwendung der in Beispiel 24 beschriebenen Methode zur Alkylierung von Sulfonamiden lassen sich z.B. folgende Verbindungen synthetisieren:
Aus 4-<4-[2-(4-Chlor-benzolsulfonylamino)ethyl]phenyl>-4-oxo-butansaeure-ethylester

1) und Methyljodid:
4-<4-[2-(N-Methyl-4-chlor-benzolsulfonylamino)ethyl]phenyl>-4-oxo-butansaeure-ethylester
Ausb. 92 %, farbloses Oel.

2) und Benzylchlorid:
4-<4-[2-(N-Benzyl-4-chlor-benzolsulfonylamino)ethyl]phenyl>-4-oxo-butansaeure-ethylester
Ausb. 85 % d.Th., Schmp. 99-100°C (Ethanol).
3) und 4-Chlor-cinnamylchlorid:
4-<4-[2-[N-(4-Chlor-cinnamyl)-4-chlor-benzolsulfonylamino]ethyl]phenyl>-4-oxo-butansaeure-ethylester
Ausb. 74 % d.Th., Schmp. 78-80°C (Ethanol).
und aus 4-Chlor-benzolsulfonsaeure-[4-(2-hydroxyethyl)phenethylamid] und 4-Chlor-cinnamylchlorid:
4) 4-Chlor-benzolsulfonsaeure-[N-(4-chlorcinnamyl)-4-(2-hydroxyethyl]phenethylamid
Ausb. 71 % d.Th., Schmp. 100-101°C (Toluol).

**Patentansprüche**

1. Sulfonyl-phenylalkylamine der Formel II

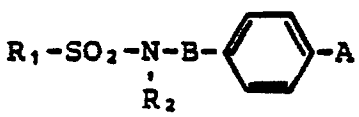

(II),

in welcher
$R_1$ eine niedere Alkylgruppe mit 1–6 C-Atomen, einen Cycloalkylrest mit 3–7 C-Atomen, einen Aralkyl-, Aralkenyl- oder Arylrest, wobei der Arylteil jeweils ein- oder mehrfach durch Halogen, $C_1$–$C_6$ Alkyl, $C_1$–$C_6$ Alkoxy, Hydroxyl, Trifluormethyl, Cyan, Nitro, Amino, $C_1$–$C_6$ Alkylamino, $C_2$–$C_{12}$ Dialkylamino, $C_1$–$C_6$ Acylamino, $C_1$–$C_{16}$ Acyl oder Azid substituiert sein kann,
$R_2$ ein Wasserstoffatom, eine Alkylgruppe mit 1–6 C-Atomen, eine Acylgruppe, einen Aralkyl- oder Aralkenylrest darstellt, wobei der Arylteil jeweils ein- oder mehrfach durch Halogen, $C_1$–$C_6$ Alkyl, $C_1$–$C_6$ Alkoxy, Hydroxyl, Trifluormethyl, Cyan, Nitro, Amino, $C_1$–$C_6$ Alkylamino, $C_2$–$C_{12}$ Dialkylamino, $C_1$–$C_6$ Acylamino, $C_1$–$C_{16}$ Acyl oder Azid substituiert sein kann,
B eine unverzweigte oder verzweigte Alkylenkette mit maximal 4 C-Atomen und
A eine Alkyl- oder Alkenylgruppe mit 1–6 C-Atomen, eine $C_1$–$C_6$ Formylalkylgruppe, eine $C_1$–$C_6$ Hydroxyalkylgruppe oder einen Rest –D–$R_3$ darstellt, in dem D eine –CO– oder –CHOH– Gruppe und $R_3$ Wasserstoff, einen $C_1$–$C_5$ Alkyl-, einen $C_1$–$C_5$ Hydroxyalkyl- oder einen $C_1$–$C_5$ Alkylcarbonsäure-Rest bedeuten,
darstellen, sowie deren pharmakologisch verträgliche Salze, und deren Ester, Amide und Lactone, sofern diese Verbindungen eine Carboxylgruppe oder eine Hydroxy- und Carboxylgruppe enthalten, mit der Maßgabe, daß für den Fall, daß $R_2$ ein Wasserstoffatom oder eine $C_1$–$C_5$-Alkylgruppe bedeutet und B eine geradkettige $C_1$–$C_3$-Alkylenkette darstellt, A dann nur eine Alkyl- oder Alkenylgruppe mit 1–6 C-Atomen sein kann.

2. Verbindungen gemäß Anspruch 1 der allgemeinen Formel II, in welcher
$R_1$ eine Methyl-, Ethyl- oder n-Hexylgruppe, einen Cyclohexylrest, einen Phenethyl- oder Styrylrest, in denen der Phenylteil durch Halogen substituiert sein kann, einen Phenylrest, der ggf. durch Halogen, Methyl, i-Propyl, Trifluormethyl, Methoxy, Hydroxy, Cyano, Nitro, Azid, Acetyl oder Acetylamino substituiert ist, eine Naphthylgruppe oder eine Biphenylgruppe, die durch Halogen substituiert sein kann, darstellt,
$R_2$ Wasserstoff, eine Methyl-, Acetyl-, Octanoyl-, Hexadecanoylgruppe, einen ggf. durch Halogen substituierten Benzoylrest, oder eine Benzyl-, Phenethyl- und Cinnamylgruppe, deren Phenylteil durch Halogen substituiert sein kann, bedeutet,
B eine Methylen-, Ethylen- oder Propylengruppe ist und
A eine Ethyl-, Propyl-, Butyl- oder Pentylgruppe, die ggf. ein- oder zweifach durch Hydroxy, Carboxyl, Formyl, Acetyloxy und Benzoyloxy substituiert ist, eine Hydroxymethyl-, Formyl-, Propenyl-, Acetylvinylgruppe oder einen Acetyl-, Propionyl- und Butyrylrest, die jeweils durch Hydroxy, Carboxy, Ethoxycarbonyl und Methoxycarbonyl substituiert sein können, darstellt,
sowie deren Ester, Amide oder Lactone, sofern diese Verbindungen eine Carboxylgruppe oder eine Hydroxy- und Carboxylgruppe enthalten.

3. Verfahren zur Herstellung von Verbindungen der Formel II

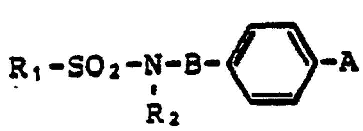

(II),

in welcher
$R_1$ eine niedere Alkylgruppe mit 1–6 C-Atomen, einen Cycloalkylrest mit 3–7 C-Atomen, einen Aralkyl-, Aralkenyl- oder Arylrest, wobei der Arylteil jeweils ein- oder mehrfach durch Halogen, $C_1$–$C_6$ Alkyl, $C_1$–

$C_6$ Alkoxy, Hydroxyl, Trifluormethyl, Cyan, Nitro, Amino, $C_1$–$C_6$ Alkylamino, $C_2$–$C_{12}$ Dialkylamino, $C_1$–$C_6$ Acylamino, $C_1$–$C_{16}$ Acyl oder Azid substituiert sein kann,
$R_2$ ein Wasserstoffatom, eine Alkylgruppe mit 1–6 C-Atomen, eine Acylgruppe, einen Aralkyl- oder Aralkenylrest darstellt, wobei der Arylteil jeweils ein- oder mehrfach durch Halogen, $C_1$–$C_6$ Alkyl, $C_1$–$C_6$ Alkoxy, Hydroxyl, Trifluormethyl, Cyan, Nitro, Amino, $C_1$–$C_6$ Alkylamino, $C_2$–$C_{12}$ Dialkylamino, $C_1$–$C_6$ Acylamino, $C_1$–$C_{16}$ Acyl oder Azid substituiert sein kann,
B eine unverzweigte oder verzweigte Alkylenkette mit maximal 4 C-Atomen und
A eine Alkyl- oder Alkenylgruppe mit 1–6 C-Atomen, eine $C_1$–$C_6$ Formylalkylgruppe, eine $C_1$–$C_6$ Hydroxyalkylgruppe oder einen Rest –D–$R_3$ darstellt, in dem D eine –CO– oder –CHOH– Gruppe und $R_3$ Wasserstoff, einen $C_1$–$C_5$ Alkyl-, einen $C_1$–$C_5$ Hydroxyalkyl- oder einen $C_1$–$C_5$ Alkylcarbonsäure-Rest bedeuten,
darstellen, sowie deren pharmakologisch verträgliche Salze, und deren Ester, Amide und Lactone, sofern diese Verbindungen eine Carboxylgruppe oder eine Hydroxy- und Carboxylgruppe enthalten, mit der Maßgabe, daß für den Fall, daß $R_2$ ein Wasserstoffatom oder eine $C_1$–$C_5$-Alkylgruppe bedeutet und B eine geradkettige $C_1$–$C_3$-Alkylenkette darstellt, A dann nur einen Alkyl- oder Alkenylgruppe mit 1–6 C-Atomen sein kann,
dadurch gekennzeichnet, daß man

a) ein Amin der allgemeinen Formel III oder eines seiner Salze

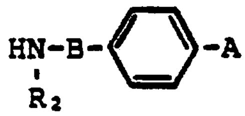 (III),

in welcher $R_2$, B und A die oben angegebene Bedeutung haben,
in an sich bekannter Weise mit einer Sulfonsäure der allgemeinen Formel IV

$R_1$–$SO_2OH$ (IV),

in welcher $R_1$ hier und in allen folgenden Beispielen die oben angegebene Bedeutung hat, bzw. einem Derivat derselben umsetzt
oder

b) ein Sulfonamid der allgemeinen Formel (V)

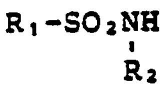 (V),

in der $R_1$ und $R_2$ die angegebene Bedeutung haben,
mit einer Verbindung der allgemeinen Formel VI

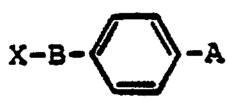 (VI),

in der X eine reaktive Gruppe darstellt,
zur Umsetzung bringt
und anschließend gewünschtenfalls erhaltene Verbindungen der Formel II in andere Verbindungen der Formel II sowie gewünschtenfalls diese Verbindungen in pharmakologisch unbedenkliche Salze überführt.

4. Verfahren zur Herstellung von Verbindungen gemäß der Formel II gemäß Anspruch 3, in der
$R_1$ eine Methyl-, Ethyl- oder n-Hexylgruppe, einen Cyclohexylrest, einen Phenethyl- oder Styrylrest, in denen der Phenylteil durch Halogen substituiert sein kann, einen Phenylrest, der ggf. durch Halogen, Methyl, i-Propyl, Trifluormethyl, Methoxy, Hydroxy, Cyano, Nitro, Azid, Acetyl oder Acetylamino substituiert ist, eine Naphthylgruppe oder eine Biphenylgruppe, die durch Halogen substituiert sein kann, darstellt,
$R_2$ Wasserstoff, eine Methyl-, Acetyl-, Octanoyl-, Hexadecanoylgruppe, einen ggf. durch Halogen substituierten Benzoylrest, oder eine Benzyl-, Phenethyl- und Cinnamylgruppe, deren Phenylteil durch Halogen substituiert sein kann, bedeutet,
B eine Methylen-, Ethylen- oder Propylengruppe ist und
A eine Ethyl-, Propyl-, Butyl- oder Pentylgruppe, die ggf. ein- oder zweifach durch Hydroxy, Carboxyl, Formyl, Acetyloxy und Benzoyloxy substituiert ist, eine Hydroxymethyl-, Formyl-, Propenyl-, Acetylvinylgruppe oder einen Acetyl-, Propionyl- und Butyrylrest, die jeweils durch Hydroxy, Carboxy, Ethoxycarbonyl und Methoxycarbonyl substituiert sein können, darstellt,

sowie deren Ester, Amide oder Lactone, sofern diese Verbindungen eine Carboxylgruppe oder eine Hydroxy- und Carboxylgruppe enthalten.

5. Arzneimittel, enthaltend mindestens eine Verbindung der allgemeinen Formel II gemäß Anspruch 1 oder 2 und ggf. pharmakologische Träger- und/oder Hilfsstoffe.

6. Verwendung von Verbindungen der allgemeinen Formel II gemäß Anspruch 1 oder 2 zur Herstellung von Arzneimitteln zur Behandlung von Krankheiten, die auf einer erhöhten Thrombozytenaggregation oder auf einer pathologischen Veränderung der Nierenfunktion beruhen.

7. Arzneimittel enthaltend mindestens eine Verbindung der allgemeinen Formel II

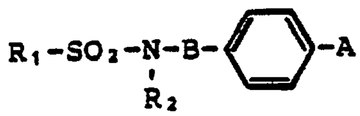

(II),

in der
$R_1$ eine niedere Alkylgruppe mit 1–6 C-Atomen, einen Cycloalkylrest mit 3–7 C-Atomen, einen Aralkyl-, Aralkenyl- oder Arylrest, wobei der Arylteil jeweils ein- oder mehrfach durch Halogen, $C_1$–$C_6$ Alkyl, $C_1$–$C_6$ Alkoxy, Hydroxyl, Trifluormethyl, Cyan, Nitro, Amino, $C_1$–$C_6$ Alkylamino, $C_2$–$C_{12}$ Dialkylamino, $C_1$–$C_6$ Acylamino, $C_1$–$C_{16}$ Acyl oder Azid substituiert sein kann,
$R_2$ ein Wasserstoffatom, eine Alkylgruppe mit 1–6 C-Atomen, eine Acylgruppe, einen Aralkyl- oder Aralkenylrest darstellt, wobei der Arylteil jeweils ein- oder mehrfach durch Halogen, $C_1$–$C_6$ Alkyl, $C_1$–$C_6$ Alkoxy, Hydroxyl, Trifluormethyl, Cyan, Nitro, Amino, $C_1$–$C_6$ Alkylamino, $C_2$–$C_{12}$ Dialkylamino, $C_1$–$C_6$ Acylamino, $C_1$–$C_{16}$ Acyl oder Azid substituiert sein kann,
B eine unverzweigte oder verzweigte Alkylenkette mit maximal 4 C-Atomen und
A eine Alkyl- oder Alkenylgruppe mit 1–6 C-Atomen, eine $C_1$–$C_6$ Formylalkylgruppe, eine $C_1$–$C_6$ Hydroxyalkylgruppe oder einen Rest –D–$R_3$ darstellt, in dem D eine –CO– oder –CHOH– Gruppe und $R_3$ Wasserstoff, einen $C_1$–$C_5$ Alkyl-, einen $C_1$–$C_5$ Hydroxyalkyl- oder einen $C_1$–$C_5$ Alkylcarbonsäure-Rest bedeuten,
darstellen, sowie deren pharmakologisch verträgliche Salze, und deren Ester, Amide und Lactone, sofern diese Verbindungen eine Carboxylgruppe oder eine Hydroxy- und Carboxylgruppe enthalten, mit Ausnahme der Verbindung 3-[4-(2-Benzolsulfonylaminoethyl)-benzoyl]propionsäure.

8. Verwendung von Verbindungen der allgemeinen Formel II gemäß Anspruch 7 zur Herstellung von Arzneimitteln zur Behandlung von Krankheiten, die auf einer erhöhten Thrombozytenaggregation oder auf einer pathologischen Veränderung der Nierenfunktion beruhen.

## Claims

1. Sulphonylphenylalkylamines of the formula II

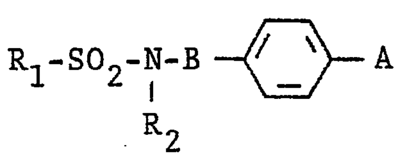

(II)

in which $R_1$ represents an alkyl group with 1–6 C-atoms, a cycloalkyl radical with 3–7 C-atoms, an aralkyl, aralkenyl or aryl radical, whereby the aryl moiety can, in each case, be substituted one or more times by halogen, $C_1$–$C_6$-alkyl, $C_1$–$C_6$-alkoxy, hydroxyl, trifluoromethyl, cyano, nitro, amino, $C_1$–$C_6$-alkylamino, $C_2$–$C_{12}$-dialkylamino, $C_1$–$C_6$-acylamino, $C_1$–$C_{16}$-acyl or azide, $R_2$ represents a hydrogen atom, an alkyl group with 1–6 C-atoms, an acyl group, an aralkyl or aralkenyl radical, whereby the aryl moiety can, in each case, be substituted one or more times by halogen, $C_1$–$C_6$-alkyl, $C_1$–$C_6$-alkoxy, hydroxyl, trifluoromethyl, cyano, nitro, amino, $C_1$–$C_6$-alkylamino, $C_2$–$C_{12}$-dialkylamino, $C_1$–$C_6$-acylamino, $C_1$–$C_{16}$-acyl or azide, B an unbranched or branched alkylene chain with a maximum of 4 C-atoms and A represents an alkyl or alkenyl group with 1–6 C-atoms, a $C_1$–$C_6$-formylalkyl radical, a $C_1$–$C_6$-hydroxyalkyl group or a radical –D–$R_3$, in which D signifies a –CO– or –CHOH– group and $R_3$ hydrogen, a $C_1$–$C_5$-alkyl, a $C_1$–$C_5$-hydroxyalkyl or a $C_1$–$C_5$-alkylcarboxylic acid radical, as well as their pharmacologically acceptable salts and their esters, amides and lactones, insofar as these compounds contain a carboxyl group or a hydroxyl and a carboxyl group, with the proviso that for the case that $R_2$ signifies a hydrogen atom or a $C_1$–$C_5$-alkyl group and B represents a straight-chained $C_1$–$C_3$-alkylene chain A can then only be an alkyl or alkenyl group with 1–6 C-atoms.

2. Compounds of the general formula II, in which $R_1$ represents a methyl, ethyl or n-hexyl group, a cyclohexyl radical, a phenethyl or styryl radical, in which the phenyl moiety can be substituted by halogen, a phenyl radical which is possibly substituted by halogen, methyl, i-propyl, trifluoromethyl, methoxy, hydroxyl, cyano, nitro, azido, acetyl or acetylamino, or a naphthyl group or a biphenyl group which can be substituted by halogen, $R_2$ signifies hydrogen, a methyl, acetyl, octanoyl, hexadecanoyl group, a benzoyl radical possibly substituted by halogen, or a benzyl, phenethyl or cinnamyl group, the phenyl moiety of which can be substituted by halogen, B is a methylene, ethylene or propylene group and A represents an ethyl, propyl, butyl or pentyl group which is possibly substituted once or twice by hydroxyl, carboxyl, formyl, acetoxy and benzoyloxy, a hydroxymethyl, formyl, propenyl, acetylvinyl group or an acetyl, propionyl and butyryl radical which, in each case, can be substituted by hydroxyl, carboxyl, ethoxycarbonyl and methoxycarbonyl, as well as their esters, amides or lactones, insofar as these compounds contain a carboxyl group or a hydroxyl and carboxyl group.

3. Process for the preparation of compounds of the formula II

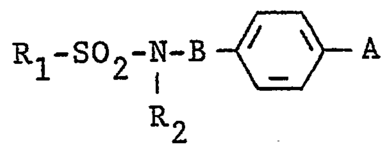 (II)

in which $R_1$ represents an alkyl group with 1–6 C-atoms, a cycloalkyl radical with 3–7 C-atoms, an aralkyl, aralkenyl or aryl radical, whereby the aryl moiety can, in each case, be substituted one or more times by halogen, $C_1$–$C_6$-alkyl, $C_1$–$C_6$-alkoxy, hydroxyl, trifluoromethyl, cyano, nitro, amino, $C_1$–$C_6$-alkylamino, $C_2$–$C_{12}$-dialkylamino, $C_1$–$C_6$-acylamino, $C_1$–$C_{16}$-acyl or azide, $R_2$ represents a hydrogen atom, an alkyl group with 1–6 C-atoms, an acyl group, an aralkyl or aralkenyl radical, whereby the aryl moiety can, in each case, be substituted one or more times by halogen, $C_1$–$C_6$-alkyl, $C_1$–$C_6$-alkoxy, hydroxyl, trifluoromethyl, cyano, nitro, amino, $C_1$–$C_6$-alkylamino, $C_2$–$C_{12}$-dialkylamino, $C_1$–$C_6$-acylamino, $C_1$–$C_{16}$-acyl or azide, B an unbranched or branched alkylene chain with a maximum of 4 C-atoms and A represents an alkyl or alkenyl group with 1–6 C-atoms, a $C_1$–$C_6$-formylalkyl group, a $C_1$–$C_6$-hydroxyalkyl group or a radical –D–$R_3$, in which D signifies a –CO– or –CHOH– group and $R_3$ hydrogen, a $C_1$–$C_5$-alkyl, a $C_1$–$C_5$-hydroxyalkyl or a $C_1$–$C_5$-alkylcarboxylic acid radical, as well as of their pharmacologically acceptable salts and of their esters, amides and lactones insofar as these compounds contain a carboxyl group or a hydroxyl and carboxyl group, with the proviso that for the case that $R_2$ signifies a hydrogen atom or a $C_1$–$C_5$-alkyl group and B represents a straight-chained $C_1$–$C_3$-alkylene chain A can then only be an alkyl or alkenyl group with 1–6 C-atoms, characterised in that one

a) reacts an amine of the general formula III or one of its salts

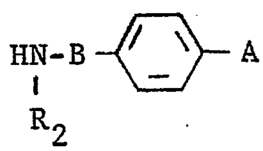 (III),

in which $R_2$, B and A have the above-given meaning, in per se known manner with a sulphonic acid of the general formula IV

$R_1$–$SO_2OH$ (IV),

in which $R_1$ and in all following examples has the above-given meaning, or a derivative thereof; or

b) brings a sulphonamide of the general formula V

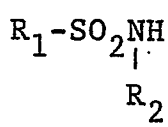 (V),

in which $R_1$ and $R_2$ have the given meaning, to reaction with a compound of the general formula VI

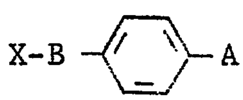 (VI),

in which X represents a reactive group, and subsequently, if desired, converts compounds obtained of the formula II into other compounds of the formula II, as well as, if desired, converts these compounds into pharmacologically acceptable salts.

4. Process for the preparation of compounds according to formula II according to claim 3, in which $R_1$ represents a methyl, ethyl or n-hexyl group, a cyclohexyl radical, a phenethyl or styryl radical in which the phenyl moiety can be substituted by halogen, a phenyl radical which is possibly substituted by halogen, methyl, i-propyl, trifluoromethyl, methoxy, hydroxyl, cyano, nitro, azido, acetyl or acetylamino, a

naphthyl group or a biphenyl group which can be substituted by halogen, $R_2$ signifies hydrogen, a methyl, acetyl, octanoyl, hexadecanoyl group, a benzoyl radical possibly substituted by halogen, or a benzyl, phenethyl and cinnamyl group the phenyl moiety of which can be substituted by halogen, B is a methylene, ethylene or propylene group and A represents an ethyl, propyl, butyl or pentyl group which is possibly substituted once or twice by hydroxyl, carboxyl, formyl, acetyloxy and benzoyloxy, a hydroxymethyl, formyl, propenyl, acetylvinyl group or an acetyl, propionyl and butyryl radical which, in each case, can be substituted by hydroxyl, carboxyl, ethoxycarbonyl and methoxycarbonyl, as well as of their esters, amides or lactones insofar as these compounds contain a carboxyl group or a hydroxyl and carboxyl group.

5. Medicaments containing at least one compound of the general formula II according to claim 1 or 2, and possibly pharmacological carrier and/or adjuvant materials.

6. Use of compounds of the general formula II according to claim 1 or 2 for the preparation of medicaments for the treatment of diseases which are due to an increased thrombocyte aggregation or to a pathological change of the kidney function.

7. Medicaments containing at least one compound of the general formula II

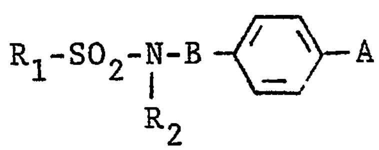

(II)

in which $R_1$ signifies an alkyl group with 1–6 C-atoms, a cycloalkyl radical with 3–7 C-atoms, an aralkyl, aralkenyl or aryl radical, whereby the aryl moiety can, in each case, be substituted one or more times by halogen, $C_1$–$C_6$-alkyl, $C_1$–$C_6$-alkoxy, hydroxyl, trifluoromethyl, cyano, nitro, amino, $C_1$–$C_6$-alkylamino, $C_2$–$C_{12}$-dialkylamino, $C_1$–$C_6$-acylamino, $C_1$–$C_{16}$-acyl or azide, $R_2$ represents a hydrogen atom, an alkyl group with 1–6 C-atoms, an acyl radical, an aralkyl or aralkenyl radical, whereby the aryl moiety can, in each case, be substituted one or more times by halogen, $C_1$–$C_6$-alkyl, $C_1$–$C_6$-alkoxy, hydroxyl, trifluoromethyl, cyano, nitro, amino, $C_1$–$C_6$-alkylamino, $C_2$–$C_{12}$-dialkylamino, $C_1$–$C_6$-acylamino, $C_1$–$C_{16}$-acyl or azide, B signifies an unbranched or branched alkylene chain with a maximum of 4 C-atoms and A signifies an alkyl or alkenyl group with 1–6 C-atoms, a $C_1$–$C_6$-formylalkyl group, a $C_1$–$C_6$-hydroxyalkyl group or a radical $-D-R_3$, in which D signifies a $-CO-$ or $-CHOH-$ group and $R_3$ hydrogen, a $C_1$–$C_5$-alkyl, a $C_1$–$C_5$-hydroxyalkyl or a $C_1$–$C_5$-alkylcarboxylic acid radical, as well as their pharmacologically acceptable salts and their esters, amides and lactones insofar as these compounds contain a carboxyl group or a hydroxyl and a carboxyl group, with the exception of the compound 3-[4-(2-benzenesulphonylaminoethyl)-benzoyl]-propionic acid.

8. Use of compounds of the general formula II according to claim 7 for the preparation of medicaments for the treatment of diseases which are due to an increased thrombocyte aggregation or to a pathological change of the kidney function.

**Revendications**

1. Sulfonyl-phénylalkylamine de formule II

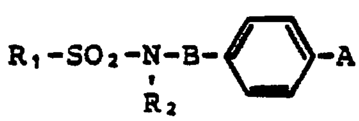

(II),

où

$R_1$ représente un groupe alkyle inférieur comportant 1 à 6 atomes de carbone, un groupe cycloalkyle comportant 3 à 7 atomes de carbone, un groupe aralkyle, aralcényle ou aryle, la partie aryle pouvant être substituée une ou plusieurs fois par un halogène, un groupe alkyle en $C_1$–$C_6$, un groupe alcoxy en $C_1$–$C_6$, un groupe hydroxyle, un groupe trifluorométhyle, un groupe cyano, nitro, amino, alkylamino en $C_1$–$C_6$, dialkylamino en $C_2$–$C_{12}$, acylamino en $C_1$–$C_6$, acyle en $C_1$–$C_{16}$ ou azide,

$R_2$ représente un atome d'hydrogène, un groupe alkyle comportant 1 à 6 atomes de carbone, un groupe acyle, un groupe aralkyle ou un groupe aralcényle, le groupe aryle pouvant être substitué une ou plusieurs fois par un halogène, un groupe alkyle en $C_1$–$C_6$, un groupe alcoxy en $C_1$–$C_6$, un groupe hydroxyle, un groupe trifluorométhyle, un groupe cyano, nitro, amino, alkylamino en $C_1$–$C_6$, dialkylamino en $C_2$–$C_{12}$, acylamino en $C_1$–$C_6$, acyle en $C_1$–$C_{16}$ ou azide,

B représente une chaîne alkylène droite ou ramifiée, avec au maximum 4 atomes de carbone, et

A représente un groupe alkyle ou alcényle comportant 1 à 6 atomes de carbone, un groupe formylalkyle en $C_1$–$C_6$, un groupe hydroxyalkyle en $C_1$–$C_6$, ou un groupe $-D-R_3$, dans lequel D représente un groupe $-CO-$ ou $-CHOH-$ et $R_3$ un atome d'hydrogène, un groupe alkyle en $C_1$–$C_5$, un groupe hydroxyalkyle en $C_1$–$C_5$ ou un groupe acide alkylcarboxylique en $C_1$–$C_5$,

ainsi que leurs sels pharmaceutiquement acceptables, et leurs esters, amides et lactones, dans la mesure où ces composés comprennent un groupe carboxyle ou un groupe hydroxy et carboxyle, étant entendu que dans le cas où $R_2$ représente un atome d'hydrogène ou un groupe alkyle en $C_1$–$C_5$, et B représente une chaîne alkylène droite en $C_1$–$C_3$, A ne peut représenter qu'un groupe alkyle ou alcényle comportant 1 à 6 atomes de carbone.

2. Composés selon la revendication 1, de formule générale II, où

$R_1$ représente un groupe méthyle, éthyle ou n-hexyle, un groupe cyclohexyle, un groupe phénéthyle ou styryle, la partie phényle pouvant être substituée par un halogène, un groupe phényle, qui peut être éventuellement substitué par un atome d'halogène, un groupe méthyle, i-propyle, trifluorométhyle, méthoxy, hydroxy, cyano, nitro, azide, acétyle ou acétylamino, un groupe naphtyle ou un groupe biphényle, qui peut être substitué par un groupe halogène,

$R_2$ représente un atome d'hydrogène, un groupe méthyle, acétyle, octanoyle, hexadécanoyle, un groupe benzoyle éventuellement substitué par un atome d'halogène, ou un groupe benzyle, phénéthyle et cinnamyle, dont la partie phényle peut être substituée par un halogène,

B représente un groupe méthylène, éthylène ou propylène, et

A représente un groupe éthyle, propyle, butyle ou pentyle, qui est éventuellement substitué une ou deux fois par un groupe hydroxy, carboxyle, formyle, acétyloxy et benzoyloxy, en groupe hydroxyméthyle, formyle, propényle, acétylvinyle ou un groupe acétyle, propionyle et butyryle, qui peut être substitué par un groupe hydroxy, carboxyle, éthoxycarbonyle et méthoxycarbonyle,

ainsi que leurs esters, amides ou lactones, dans la mesure où ces composés comprennent un groupe hydroxy ou carboxyle.

3. Procédé pour la fabrication des composés de formule II

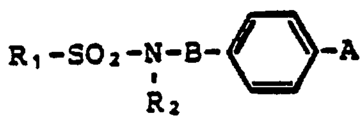

(II),

où

$R_1$ représente un groupe alkyle inférieur comportant 1 à 6 atomes de carbone, un groupe cycloalkyle comportant 3 à 7 atomes de carbone, un groupe aralkyle, aralcényle ou aryle, la partie aryle pouvant être substituée une ou plusieurs fois par un halogène, un groupe alkyle en $C_1$–$C_6$, un groupe alcoxy en $C_1$–$C_6$, un groupe hydroxyle, un groupe trifluorométhyle, un groupe cyano, nitro, amino, alkylamino en $C_1$–$C_6$, dialkylamino en $C_2$–$C_{12}$, acylamino en $C_1$–$C_6$, acyle en $C_1$–$C_{16}$ ou azide,

$R_2$ représente un atome d'hydrogène, un groupe alkyle comportant 1 à 6 atomes de carbone, un groupe acyle, un groupe aralkyle ou un groupe aralcényle, le groupe aryle pouvant être substitué une ou plusieurs fois par un halogène, un groupe alkyle en $C_1$–$C_6$, un groupe alcoxy en $C_1$–$C_6$, un groupe hydroxyle, un groupe trifluorométhyle, un groupe cyano, nitro, amino, alkylamino en $C_1$–$C_6$, dialkylamino en $C_2$–$C_{12}$, acylamino en $C_1$–$C_6$, acyle en $C_1$–$C_{16}$ ou azide,

B représente une chaîne alkylène droite ou ramifiée, avec au maximum 4 atomes de carbone, et

A représente un groupe alkyle ou alcényle comportant 1 à 6 atomes de carbone, un groupe formylalkyle en $C_1$–$C_6$, un groupe hydroxyalkyle en $C_1$–$C_6$, ou un groupe $-D-R_3$, dans lequel D représente un groupe $-CO-$ ou $-CHOH-$ et $R_3$ un atome d'hydrogène, un groupe alkyle en $C_1$–$C_5$, un groupe hydroxyalkyle en $C_1$–$C_5$ ou un groupe acide alkylcarboxylique en $C_1$–$C_5$,

ainsi que leurs sels pharmaceutiquement acceptables, et leurs esters, amides et lactones, dans la mesure où ces composés comprennent un groupe carboxyle ou un groupe hydroxy et carboxyle, étant entendu que dans le cas où $R_2$ représente un atome d'hydrogène ou un groupe alkyle en $C_1$–$C_5$, et B représente une chaîne alkylène droite en $C_1$–$C_3$, A ne peut représenter qu'un groupe alkyle ou alcényle comportant 1 à 6 atomes de carbone, caractérisé en ce que

a) on fait réagir une amine de formule générale III ou un de ses sels

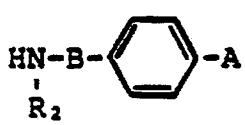

(III),

où $R_2$, B et A ont les significations mentionnées ci-dessus,

de manière connue en soi, avec un acide sulfonique de formule générale IV

$$R_1\text{–}SO_2OH \qquad (IV),$$

où $R_1$ représente, ici et dans tous les exemples suivants, la signification mentionnée plus haut, ou un de ses dérivés,

ou

b) on fait réagir un sulfonamide de formule générale V

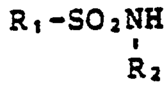 (V),

où $R_1$ et $R_2$ ont les significations mentionnées ci-dessus, avec un composé de formule générale VI

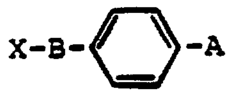 (VI),

où X représente un groupe réactif,

et ensuite, si on le souhaite, on transforme les composés de formule II obtenus en d'autres composés de formule II, et si on le souhaite, on transforme ces composés en sels pharmacologiquement acceptables.

4. Procédé pour la préparation de composés de formule II selon la revendication 3, où

$R_1$ représente un groupe méthyle, éthyle ou n-hexyle, un groupe cyclohexyle, un groupe phénéthyle ou styryle, la partie phényle pouvant être substituée par un halogène, un groupe phényle, qui peut être éventuellement substitué par un atome d'halogène, un groupe méthyle, i-propyle, trifluorométhyle, méthoxy, hydroxy, cyano, nitro, azide, acétyle ou acétylamino, un groupe naphtyle ou un groupe biphényle, qui peut être substitué par un groupe halogène,

$R_2$ représente un atome d'hydrogène, un groupe méthyle, acétyle, octanoyle, hexadécanoyle, un groupe benzoyle éventuellement substitué par un atome d'halogène, ou un groupe benzyle, phénéthyle et cinnamyle, dont la partie phényle peut être substituée par un halogène,

B représente un groupe méthylène, éthylène ou propylène, et

A représente un groupe éthyle, propyle, butyle ou pentyle, qui est éventuellement substitué une ou deux fois par un groupe hydroxy, carboxyle, formyle, acétyloxy et benzoyloxy, un groupe hydroxyméthyle, formyle, propényle, acétylvinyle ou un groupe acétyle, propionyle et butyryle, qui peut être substitué par un groupe hydroxy, carboxyle, éthoxycarbonyle et méthoxycarbonyle,

ainsi que leurs esters, amides ou lactones, dans la mesure où ces composés comprennent un groupe hydroxy ou carboxyle.

5. Médicament, contenant au moins un composé de formule générale II selon la revendication 1 ou 2, et éventuellement des substances de support et/ou des adjuvants pharmacologiques.

6. Utilisation des composés de formule générale II selon la revendication 1 ou 2, pour la préparation de médicaments pour le traitement de maladies qui sont dûes à une agglomération accrue de thrombocytes ou à une modification pathologique des fonctions reinales.

7. Médicament contenant au moins un composé de formule générale II,

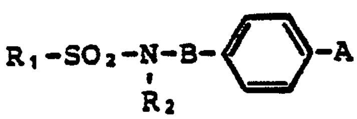 (II),

où

$R_1$ représente un groupe alkyle inférieur comportant 1 à 6 atomes de carbone, un groupe cycloalkyle comportant 3 à 7 atomes de carbone, un groupe aralkyle, aralcényle ou aryle, la partie aryle pouvant être substituée une ou plusieurs fois par un halogène, un groupe alkyle en $C_1$–$C_6$, un groupe alcoxy en $C_1$–$C_6$, un groupe hydroxyle, un groupe trifluorométhyle, un groupe cyano, nitro, amino, alkylamino en $C_1$–$C_6$, dialkylamino en $C_2$–$C_{12}$, acylamino en $C_1$–$C_6$, acyle en $C_1$–$C_{16}$ ou azide,

$R_2$ représente un atome d'hydrogène, un groupe alkyle comportant 1 à 6 atomes de carbone, un groupe acyle, un groupe aralkyle ou un groupe aralcényle, le groupe aryle pouvant être substitué une ou plusieurs fois par un halogène, un groupe alkyle en $C_1$–$C_6$, un groupe alcoxy en $C_1$–$C_6$, un groupe hydroxyle, un groupe trifluorométhyle, un groupe cyano, nitro, amino, alkylamino en $C_1$–$C_6$, dialkylamino en $C_2$–$C_{12}$, acylamino en $C_1$–$C_6$, acyle en $C_1$–$C_{16}$ ou azide,

B représente une chaîne alkylène droite ou ramifiée, avec au maximum 4 atomes de carbone, et

A représente un groupe alkyle ou alcényle comportant 1 à 6 atomes de carbone, un groupe formylalkyle en $C_1$–$C_6$, un groupe hydroxyalkyle en $C_1$–$C_6$, ou un groupe –D–$R_3$, dans lequel D représente un groupe –CO– ou –CHOH– et $R_3$ un atome d'hydrogène, un groupe alkyle en $C_1$–$C_5$, un groupe hydroxyalkyle en $C_1$–$C_5$ ou un groupe acide alkylcarboxylique en $C_1$–$C_5$,

ainsi que leurs sels pharmaceutiquement acceptables, et leurs esters, amides et lactones, dans la mesure où ces composés comprennent un groupe carboxyle ou un groupe hydroxy et carboxyle, à l'exception de l'acide 3-[4-(2-benzolsulfonylaminoéthyl)-benzoyl]propionique.

8. Utilisation des composés de formule générale II selon la revendication 7, pour la préparation de médicaments pour le traitement de maladies qui sont dûes à une agglomération accrue de thrombocytes ou à une modification pathologique des fonctions reinales.